# EUROPEAN PATENT APPLICATION

(11) **EP 4 620 621 A1**
(43) Date of publication of application: **24.09.2025**
(21) Application number: 25161295.8
(22) Date of filing: 03.03.2025
(51) Int. Cl.: B25B 13/08, B25B 13/48, B25B 23/142

(54) **TORQUE WRENCH**

(30) Priority: 22.03.2024 US 202418613317
(71) Applicant: Kogent Surgical, LLC, Chesterfield, MO 63005 (US)
(72) Inventor: Aach, Joseph E., Chesterfield, 63005 (US)
(74) Representative: Carl Zeiss AG - Patentabteilung

(57) **Abstract**

A torque wrench for connecting a first element to a second element includes a shaft extending between a first end and a second end. The torque wrench includes a first tightening socket at the first end. The first tightening socket includes a first spanner pocket configured to receive the first element to tighten the first element to the second element when the torque wrench is rotated in a tightening direction. The first tightening socket is connected to the first end of the shaft by a first weakened section configured to yield at a predetermined peak torque. The torque wrench includes a loosening socket separate from the first tightening socket. The loosening socket includes a loosening spanner pocket configured to receive the first element to loosen the first element from the second element when the torque wrench is rotated in a loosening direction.

## Description

### BACKGROUND OF THE INVENTION

The subject matter herein relates generally to torque wrenches.

Torque wrenches are known to be useful in many different applications, including in the assembly of surgical devices. For example, torque wrenches may be used to connect surgical attachments, such as different working tips, onto a handpiece, such as an ultrasonic handpiece used in removing patient tissue during a surgical procedure. Known torque wrenches for use in medicine utilize either a clutch means, a spring loading, or interacting parts movable relative to each other. However, such torque wrenches may be complex and expensive to manufacture, which increases the overall cost of the surgical tools for the medical provider. Additionally, surgical procedures require sterilization of parts used in the operating room. It is difficult to sterilize some known torque wrenches due to the complex parts used in the torque wrench. Additionally, disassembly of the torque wrenches for sterilization is time consuming. Some known torque wrenches are designed as single use torque wrenches, which are manufactured inexpensively. However, such torque wrenches provide inconsistent torque parameters leading to overtightening or under-tightening of the components, which may lead to poor performance of the surgical device. Additionally, some known single use torque wrenches are designed to fracture when the peak torque is reached, leading to fragments of wrench material being deposited on the surgical instrument, or surgical site, increasing risk to patients and users.

A need remains for a torque wrench that is compact, simply constructed, inexpensive, biocompatible, sterilizable, reliable and disposable. A need remains for a torque wrench that is easy to use and provides repeatable and consistent torque application for assembling various elements.

### BRIEF DESCRIPTION OF THE INVENTION

The subject-matter of the independent claims solves the above-mentioned problems. Advantages embodiments of the invention are subject matter of the dependent claims.

In one embodiment, a torque wrench for connecting a first element to a second element is provided. The first element could be a surgical attachment, e.g. a working element usable during a surgical procedure, especially a surgical tip configured for using in connection with an ultrasonic surgical device. For example, the surgical attachment may be used to remove soft tissue and/or fibrous tissue and/or hard tissue from a patient during a surgical procedure. The second element could be a handpiece, e.g. a surgical handpiece, especially an ultrasonic handpiece configured to generate ultrasonic motion of the surgical attachment. The handpiece may be part of an ultrasonic surgical device. Ultrasonic surgical devices are used in surgical procedures for various applications, such as, dissection, aspiration, coagulation, and cutting of biological tissue. Typically, ultrasonic surgical devices use piezoelectric transducers to operate as a half-wavelength resonator by generating a high frequency wave oscillation that vibrates various surgical tools at a resonant frequency. The vibration may be longitudinal, radial, flexural, torsional or a combination of such forms of vibration. Compared to traditional surgical tools and techniques, ultrasonic surgical devices provide more precise cutting and better coagulation of the tissue than electro-surgical instruments, thereby reducing bleeding and reduced damage to surrounding tissue. In addition, ultrasonic vibration provides for less thermal damage, such as charring, and less desiccation than cryogenic or electro-surgical instruments.

The torque wrench includes a shaft extending between a first end and a second end. The shaft forms the main body of the torque wrench which may be gripped by the user. The shaft is a generally rigid body that may be held by the user. The shaft is sized and shaped to fit in the users hand. The shaft may include ergonomic gripping features to allow ease of holding the torque wrench. The shaft positions other elements of the torque wrench relative to each other. The torque wrench includes a first tightening socket at the first end. The first tightening socket defines a space to receive the first element and to grip the first element for tightening the first element to the second element. THe tightening socket is open ended to receive the first element. The first tightening socket includes a first spanner pocket configured to receive the first element to tighten the first element to the second element when the torque wrench is rotated in a tightening direction. The spanner pocket defines a space to receive the first element. The spanner pocket is sized and shaped to interface with the first element. The spanner pocket is open to receive the first element, such as through a side of the first tightening socket. Tightening refers to applying force to the first element in a direction to connect the first element to the second element. When tightening, the force applied to the first element increases when the torque wrench is rotated until the torque wrench reaches the maximum torque and fails or yields. The first tightening socket is connected to the first end of the shaft by a first weakened section configured to yield at a predetermined torque. In the context of this application, yield can be used synonymously to fail, e.g., the first tightening socket is connected to the first end of the shaft by a first weakened section configured to fail at a predetermined torque. The first tightening socket may have a large cross-section area allowing for a high percent error in the geometry while still maintaining accurate applied torque. This enables to reach the predetermined torque in a wide range of bend deformation, i.e., the predetermined torque may be achieved with a deflection angle of about 8° to about 170° of arm deflection). The torque wrench includes a loosening socket separate from the tightening socket. The loosening socket includes a loosening spanner pocket configured to receive the first element to loosen the first element from the second element when the torque wrench is rotated in a loosening direction. Loosening refers to applying a force to the first element in a direction to disconnect the first element from the second element.

In a preferred embodiment, the torque wrench further comprises a second tightening socket at the second end, the second tightening socket including a second spanner pocket configured to receive the first element to tighten the first element to the second element when the torque wrench is rotated in a tightening direction, the second tightening socket connected to the second end of the shaft by a second weakened section configured to yield at the predetermined peak torque. This enables the user to make two tightening attempts, allowing the user to more than one attempt to properly install e.g., an ultrasonic tip to an ultrasonic handpiece while minimizing potential error and resources consumed.

In a preferred embodiment, the first and second tightening sockets are identical and face in opposite directions on the opposite first and second ends of the shaft. This ensures a reproducible tightening procedure in addition to an easy-to-use design and a simple construction.

In a preferred embodiment, the torque wrench is a disposable torque wrench with the first tightening socket being a single use torque socket unusable after the first weakened section yields. In another preferred embodiment, the torque wrench is a disposable torque wrench with the second tightening socket being a single use torque socket unusable after the second weakened section yields. In another preferred embodiment, the torque wrench is a disposable torque wrench with the first tightening socket and the second tightening socket being single use torque sockets unusable after the first weakened section and the second weakened section yield. Having a single use tightening socket unusable after yielding of the weakened section allows for a more accurate and consistent torque application and increases the reliability while reducing manufacturing costs.

In a preferred embodiment, the loosening socket is reusable. This is advantageous since the user may always loosen the first element from the second element when the torque wrench is rotated in a loosening direction. However, the loosening socket may also be disposable.

In a preferred embodiment, the first weakened section deforms at the predetermined peak torque. This ensures a more accurate and consistent torque application and increases the reliability.

In a preferred embodiment, the first weakened section deforms by bending backwards toward the shaft. This ensures a more accurate and consistent torque application and increases the reliability.

In a preferred embodiment, the first weakened section is configured to deform between a range of approximately 0° to approximately 180°, the applied torque plateauing at the predetermined peak torque through the range of deformation. This ensures a more accurate and consistent torque application across a wider range and increases the reliability.

In a preferred embodiment, the first weakened section includes a first neck having a reduced cross-sectional area compared to the shaft. In another preferred embodiment, the second weakened section includes a second neck having a reduced cross-sectional area compared to the shaft. In another preferred embodiment, first weakened section includes a first neck having a reduced cross-sectional area compared to the shaft and the second weakened section includes a second neck having a reduced cross-sectional area compared to the shaft. Having a weakened section with a neck having a reduced cross-section area compared to the shaft ensures that when applying the predetermined force, the weakened section and not the shaft will yield.

In a preferred embodiment, the first weakened section extends between the first end of the shaft and the first tightening socket. This ensures that the technical feature is responsible for tightening the first element to the second element when the torque wrench is rotated in a tightening direction, i.e., the tightening socket will not yield when the predetermined force is applied but the first weakened section spaced apart from the tightening means ensuring a safer tightening of the first element to the second element.

In a preferred embodiment, the first weakened section does not directly interface with the first element ensuring a safer tightening of the first element to the second element. In other words, the first weakened section is spaced apart from the first element ensuring a safer tightening of the first element to the second element.

In a preferred embodiment, the first tightening socket includes an upper jaw and a lower jaw on opposite sides of the first spanner pocket, the upper jaw and lower jaw of the first tightening socket having flat interface surfaces configured to engage flat interface surfaces of the first element to rotate the first element when the torque wrench is tightened. This enables a better engagement between the tightening socket and the first element.

In a preferred embodiment, the lower jaw is shorter than the upper jaw allowing rotation of the first element in only the tightening direction ensuring a safer tightening of the first element to the second element.

In a preferred embodiment, the first tightening socket includes a first keying feature configured to interface with the first element in only one orientation. In another preferred embodiment, the second tightening socket includes a second keying feature configured to interface with the first element in only one orientation. In another preferred embodiment, the first tightening socket includes a first keying feature configured to interface with the first element in only one orientation and the second tightening socket includes a second keying feature configured to interface with the first element in only one orientation. This prevents the user from putting the wrench onto the first element backwards and misusing the wrench ensuring a properly fitting of the wrench and the correct application of the torque.

In a preferred embodiment, the loosening socket includes a first jaw and a second jaw on opposite sides of the loosening spanner pocket, the first jaw and the second jaw having flat interface surfaces configured to engage flat interface surfaces of the first element to rotate the first element when the torque wrench is loosened. This enables a better engagement between the loosening socket and the first element.

In a preferred embodiment, the first tightening socket faces in a first direction and the loosening socket faces in a second direction perpendicular to the first direction. In another preferred embodiment, the second tightening socket faces in a first direction and the loosening socket faces in a second direction perpendicular to the first direction. In another preferred embodiment, the first tightening socket faces in a first direction, the second tightening socket faces in a second direction parallel to the first direction and the loosening socket faces in a third direction perpendicular to the first and second direction. The sockets face in different directions to allow positioning of the sockets at different areas of the torque wrench, such as at different ends or sides of the torque wrench. The sockets face in different directions for manufacturability. The sockets face in different directions for a compact design.

In a preferred embodiment, the first tightening socket and the loosening socket are integral with the shaft to define a single piece torque wrench. In another preferred embodiment, the second tightening socket and the loosening socket are integral with the shaft to define a single piece torque wrench. In another preferred embodiment, the first tightening socket, second tightening socket and the loosening socket are integral with the shaft to define a single piece torque wrench ensuring low manufacturing costs for the torque wrench.

In a preferred embodiment, the shaft is manufactured from a metal material being sterilizable. In another preferred embodiment, the shaft is manufactured from a metal material being biocompatible. In another preferred embodiment, the shaft is manufactured from a metal material being disposable. In another preferred embodiment, the shaft is manufactured from a metal material being sterilizable, biocompatible and disposable. In another preferred embodiment, each combination of these features is realizable.

In another embodiment, a torque wrench for connecting a first element to a second element is provided. The first element could be a surgical attachment, e.g., a surgical tip especially configured for using in connection with an ultrasonic surgical device. The second element could be a handpiece, especially a surgical handpiece. The handpiece may be part of an ultrasonic surgical device. The torque wrench includes a shaft extending between a first end and a second end. The shaft forms the main body of the torque wrench which may be gripped by the user. The shaft is a generally rigid body that may be held by the user. The shaft is sized and shaped to fit in the users hand. The shaft may include ergonomic gripping features to allow ease of holding the torque wrench. The shaft positions other elements of the torque wrench relative to each other. The torque wrench includes a first tightening socket including a first spanner pocket configured to receive the first element to tighten the first element to the second element when the torque wrench is rotated in a tightening direction. The first tightening socket defines a space to receive the first element and to grip the first element for tightening the first element to the second element. The first tightening socket is open ended to receive the first element. Socket in this application is referring to the part the wrench designed to engage with the part torque is being applied to. The spanner pocket defines a space to receive the first element. The spanner pocket is sized and shaped to interface with the first element. The spanner pocket is open to receive the first element, such as through a side of the first tightening socket. Tightening refers to applying force to the first element in a direction to connect the first element to the second element. When tightening, the force applied to the first element increases when the torque wrench is rotated until the torque wrench reaches the maximum torque and fails or yields. The first tightening socket connected to the first end of the shaft by a first weakened section configured to yield at a predetermined peak torque. In the context of this application, yield can be used synonymously to fail, e.g., the first tightening socket is connected to the first end of the shaft by a first weakened section configured to fail at a predetermined torque. The first tightening socket may have a large cross-section area allowing for a high percent error in the geometry while still maintaining accurate applied torque. This enables to reach the predetermined torque in a wide range of bend deformation, i.e., the predetermined torque may be achieved with a deflection angle of about 8° to about 170° of arm deflection). The torque wrench includes a second tightening socket includes a second spanner pocket configured to receive the first element to tighten the first element to the second element when the torque wrench is rotated in a tightening direction. Tightening refers to applying force to the first element in a direction to connect the first element to the second element. When tightening, the force applied to the first element increases when the torque wrench is rotated until the torque wrench reaches the maximum torque and fails or yields. The second tightening socket connected to the second end of the shaft by a second weakened section configured to yield at the peak torque. The second tightening socket may have a large cross-section area allowing for a high percent error in the geometry while still maintaining accurate applied torque. This enables to reach the predetermined torque in a wide range of bend deformation, i.e., the predetermined torque may be achieved with a deflection angle of about 8° to about 170° of arm deflection).

In a preferred embodiment, the first and second tightening sockets are identical and face in opposite directions on the opposite first and second ends of the shaft. This ensures a reproducible tightening procedure in addition to an easy-to-use design and a simple construction.

In a preferred embodiment, the torque wrench is a disposable torque wrench with the first tightening socket being a single use torque socket unusable after the first weakened section yields. In another preferred embodiment, the torque wrench is a disposable torque wrench with the second tightening socket being a single use torque socket unusable after the second weakened section yields. In another preferred embodiment, the torque wrench is a dual use disposable torque wrench with the first tightening socket being a single use torque socket unusable after the first weakened section yields and with the second tightening socket being a single use torque socket being unusable after the second weakened section yields. Having a single use tightening socket unusable after yielding of the weakened section allows for a more accurate and consistent torque application and increases the reliability while reducing manufacturing costs.

In a preferred embodiment, the loosening socket is reusable. This is advantageous since the user may always loosen the first element from the second element when the torque wrench is rotated in a loosening direction. However, the loosening socket may also be disposable.

In a preferred embodiment, the first tightening socket and the second tightening socket are used in different tightening operations to tighten the first element to the second element at different times. This enables the user to make two tightening attempts, allowing the user to more than one attempt to properly install e.g., an ultrasonic tip to an ultrasonic handpiece while minimizing potential error and resources consumed.

In a preferred embodiment, the first weakened section includes a first neck having a reduced cross-sectional area compared to the shaft. In another preferred embodiment, the second weakened section includes a second neck having a reduced cross-sectional area compared to the shaft. In another preferred embodiment, first weakened section includes a first neck having a reduced cross-sectional area compared to the shaft and the second weakened section includes a second neck having a reduced cross-sectional area compared to the shaft. In a preferred embodiment, the reduced cross-sectional area of the second neck is equivalent to the reduced cross-sectional area of the first neck. Having a weakened section with a neck having a reduced cross-section area compared to the shaft ensures that when applying the predetermined force, the weakened section and not the shaft will yield.

In a preferred embodiment, the first tightening socket includes an upper jaw and a lower jaw on opposite sides of the first spanner pocket, the upper jaw and the lower jaw of the first tightening socket having flat interface surfaces configured to engage flat interface surfaces of the first element to rotate the first element when the torque wrench is tightened. In another preferred embodiment, the second tightening socket includes an upper jaw and a lower jaw on opposite sides of the second spanner pocket, the upper jaw and the lower jaw of the second tightening socket having flat interface surfaces configured to engage flat interface surfaces of the first element to rotate the first element when the torque wrench is tightened. In another preferred embodiment, the first tightening socket includes an upper jaw and a lower jaw on opposite sides of the first spanner pocket, the upper jaw and the lower jaw of the first tightening socket having flat interface surfaces configured to engage flat interface surfaces of the first element to rotate the first element when the torque wrench is tightened and the second tightening socket includes an upper jaw and a lower jaw on opposite sides of the second spanner pocket, the upper jaw and the lower jaw of the second tightening socket having flat interface surfaces configured to engage flat interface surfaces of the first element to rotate the first element when the torque wrench is tightened. This enables a better engagement between the tightening sockets and the first element.

In a preferred embodiment, the first tightening socket includes a first keying feature configured to interface with the first element in only one orientation. In another preferred embodiment, the second tightening socket includes a second keying feature configured to interface with the first element in only one orientation. In another preferred embodiment, the first tightening socket includes a first keying feature configured to interface with the first element in only one orientation and the second tightening socket includes a second keying feature configured to interface with the first element in only one orientation. This prevents the user from putting the wrench onto the first element backwards and misusing the wrench ensuring a properly fitting of the wrench and the correct application of the torque.

In a preferred embodiment, the torque wrench further comprises a loosening socket along the shaft, the loosening socket including a loosening spanner pocket configured to receive the first element to loosen the first element from the second element when the torque wrench is rotated in a loosening direction. Loosening refers to applying a force to the first element in a direction to disconnect the first element from the second element. This ensures that the first element and the second element always can be separated resulting in a safe connection of the first element and the second element.

In a further embodiment, a surgical device kit is provided and includes a surgical attachment extending between a proximal end and a distal end. The surgical attachment could be e.g., a surgical tip especially configured for using in connection with an ultrasonic surgical device. The proximal end is configured to be removably coupled to a handpiece, especially a surgical handpiece. The distal end has a working element for performing a surgical operation. The working element is used to interface with the patient. The working element includes features to engage the patient and perform a surgical procedure. For example, the working element may include cutting elements to cut patient tissue. The surgical attachment includes a torque wrench interface. The torque wrench interface is the portion of the surgical attachment configured to interface with the torque wrench. For example, the torque wrench interface includes surfaces or features configured to be engaged by the torque wrench for tightening the surgical attachment to the surgical handpiece. The surgical device kit includes a torque wrench configured to interface with the surgical attachment at the torque wrench interface for tightening the surgical attachment to the handpiece to a predetermined peak torque and for loosening the surgical attachment from the handpiece. Tightening refers to applying force to the first element in a direction to connect the first element to the second element. When tightening, the force applied to the first element increases when the torque wrench is rotated until the torque wrench reaches the maximum torque and fails or yields. Loosening refers to applying a force to the first element in a direction to disconnect the first element from the second element. The torque wrench includes a shaft extending between a first end and a second end and a first tightening socket at the first end. The shaft forms the main body of the torque wrench which may be gripped by the user. The shaft is a generally rigid body that may be held by the user. The shaft is sized and shaped to fit in the users hand. The shaft may include ergonomic gripping features to allow ease of holding the torque wrench. The shaft positions other elements of the torque wrench relative to each other. The first tightening socket defines a space to receive the first element and to grip the first element for tightening the first element to the second element. The first tightening socket is open ended to receive the first element. Socket in this application is referring to the part the wrench designed to engage with the part torque is being applied to. Tightening refers to applying force to the first element in a direction to connect the first element to the second element. When tightening, the force applied to the first element increases when the torque wrench is rotated until the torque wrench reaches the maximum torque and fails or yields. The first tightening socket includes a first spanner pocket configured to receive the surgical attachment at the torque wrench interface to tighten the surgical attachment to the handpiece when the torque wrench is rotated in a tightening direction. The spanner pocket defines a space to receive the first element. The spanner pocket is sized and shaped to interface with the first element. The spanner pocket is open to receive the first element, such as through a side of the first tightening socket. The first tightening socket connected to the first end of the shaft by a first weakened section is configured to yield at the peak torque. The first tightening socket may have a large cross-section area allowing for a high percent error in the geometry while still maintaining accurate applied torque. This enables to reach the predetermined torque in a wide range of bend deformation, i.e., the predetermined torque may be achieved with a deflection angle of about 8° to about 170° of arm deflection). The loosening socket is separate from the first tightening socket. The loosening socket includes a loosening spanner pocket configured to receive the surgical attachment at the torque wrench interface to loosen the surgical attachment from the handpiece when the torque wrench is rotated in a loosening direction.

In a preferred embodiment, the torque wrench further comprises a second tightening socket at the second end, the second tightening socket including a second spanner pocket configured to receive the first element to tighten the first element to the second element when the torque wrench is rotated in a tightening direction, the second tightening socket connected to the second end of the shaft by a second weakened section configured to yield at the predetermined peak torque. This enables the user to make two tightening attempts, allowing the user to more than one attempt to properly install e.g., an ultrasonic tip to an ultrasonic handpiece while minimizing potential error and resources consumed.

In a preferred embodiment, the surgical device kit further comprises a sleeve configured to surround the surgical attachment, the sleeve configured to be coupled to the handpiece. By this, an appropriate grip is ensured protecting the user and may provide an irrigation route.

In a preferred embodiment, the first tightening socket includes a first keying feature configured to interface with the first element in only one orientation. In another preferred embodiment, the second tightening socket includes a second keying feature configured to interface with the first element in only one orientation. In another preferred embodiment, the first tightening socket includes a first keying feature configured to interface with the first element in only one orientation and the second tightening socket includes a second keying feature configured to interface with the first element in only one orientation. This prevents the user from putting the wrench onto the first element backwards and misusing the wrench ensuring a properly fitting of the wrench and the correct application of the torque.

In a further embodiment, a method of attaching a first element to a second element is provided. The first element could be a surgical attachment, e.g. a surgical tip especially configured for using in connection with an ultrasonic surgical device. The second element could be a handpiece, especially a surgical handpiece. The handpiece may be part of an ultrasonic surgical device. The method includes providing a torque wrench including a shaft extending between a first end and a second end, a first tightening socket at the first end, and a loosening socket separate from the first tightening socket. The shaft forms the main body of the torque wrench which may be gripped by the user. The shaft is a generally rigid body that may be held by the user. The shaft is sized and shaped to fit in the users hand. The shaft may include ergonomic gripping features to allow ease of holding the torque wrench. The shaft positions other elements of the torque wrench relative to each other. The first tightening socket defines a space to receive the first element and to grip the first element for tightening the first element to the second element. The first tightening socket is open ended to receive the first element. Socket in this application is referring to the part the wrench designed to engage with the part torque is being applied to. The first tightening socket includes a first spanner pocket configured to receive the first element. The spanner pocket defines a space to receive the first element. The spanner pocket is sized and shaped to interface with the first element. The spanner pocket is open to receive the first element, such as through a side of the first tightening socket. The first tightening socket connected to the first end of the shaft by a first weakened section configured to yield at a predetermined peak torque. **In** the context of this application, yield can be used synonymously to fail, e.g., the first tightening socket is connected to the first end of the shaft by a first weakened section configured to fail at a predetermined torque. The first tightening socket may have a large cross-section area allowing for a high percent error in the geometry while still maintaining accurate applied torque. This enables to reach the predetermined torque in a wide range of bend deformation, i.e., the predetermined torque may be achieved with a deflection angle of about 8° to about 170° of arm deflection). The loosening socket includes a loosening spanner pocket configured to receive the first element. The method couples the first tightening socket to the first element. The method rotates the torque wrench in a tightening direction to tighten the first element to the second element using the first tightening socket, wherein the torque wrench yields at the first weakening section when the first element is tightened to the peak torque. Tightening refers to applying force to the first element in a direction to connect the first element to the second element. When tightening, the force applied to the first element increases when the torque wrench is rotated until the torque wrench reaches the maximum torque and fails or yields. The method couples the loosening socket to the first element. The method rotates the torque wrench in a loosening direction to loosen the first element from the second element using the loosening socket. Loosening refers to applying a force to the first element in a direction to disconnect the first element from the second element.

**In** a preferred embodiment, coupling the first tightening socket to the first element includes aligning a first keyed interface of the first tightening socket with a keyed interface of the first element. This prevents the user from putting the wrench onto the first element backwards and misusing the wrench ensuring a properly fitting of the wrench and the correct application of the torque.

In a preferred embodiment, the first tightening socket includes an upper jaw and a lower jaw on opposite sides of the first spanner pocket, the upper jaw and lower jaw of the first tightening socket having flat interface surfaces, said coupling the first tightening socket to the first element includes engaging the flat interface surfaces of the upper jaw and lower jaw to flat interface surfaces of the first element to rotate the first element when the torque wrench is tightened. This enables a better engagement between the tightening socket and the first element.

In a preferred embodiment, the provided torque wrench includes a second tightening socket at the second end of the shaft, the second tightening socket includes a second spanner pocket configured to receive the first element to tighten the first element to the second element, the second tightening socket connected to the second end of the shaft by a second weakened section configured to yield at the predetermined peak torque, wherein the second tightening socket allows for a second tightening operation by the torque wrench prior to disposal of the torque wrench. This enables the user to make two tightening attempts, allowing the user to more than one attempt to properly install e.g., an ultrasonic tip to an ultrasonic handpiece while minimizing potential error and resources consumed.

In a preferred embodiment, the method further couples the second tightening socket to the first element; and rotates the torque wrench in a tightening direction to tighten the first element to the second element using the second tightening socket, wherein the torque wrench yields at the second weakening section when the first element is tightened to the predetermined peak torque. This enables the user to make two tightening attempts, allowing the user to more than one attempt to properly install e.g., an ultrasonic tip to an ultrasonic handpiece while minimizing potential error and resources consumed.

### BRIEF DESCRIPTION OF THE DRAWINGS

Figure 1 illustrates a torque wrench in accordance with an exemplary embodiment used for tightening and loosening a first element relative to a second element.
Figure 2 is an enlarged view of the torque wrench coupled to the first element for tightening the first element to the second element in accordance with an exemplary embodiment.
Figure 3 illustrates the torque wrench coupled to the first element loosening the first element from the second element in accordance with an exemplary embodiment.
Figure 4 is an exploded view of a surgical device that may be assembled using the first and second elements in accordance with an exemplary embodiment.
Figure 5 shows the first element poised for coupling to the second element in accordance with an exemplary embodiment.
Figure 6 illustrates the torque wrench in accordance with an exemplary embodiment.
Figure 7 illustrates the torque wrench in accordance with an exemplary embodiment.
Figure 8 illustrates the torque wrench in accordance with an exemplary embodiment.
Figure 9 illustrates the torque wrench in accordance with an exemplary embodiment.
Figure 10 illustrates the torque wrench in accordance with an exemplary embodiment.
Figure 11 illustrates the torque wrench in accordance with an exemplary embodiment.
Figure 12 illustrates the torque wrench in accordance with an exemplary embodiment.
Figure 13 is a top perspective view of a portion of the torque wrench showing the first tightening socket in accordance with an exemplary embodiment.
Figure 14 is a bottom perspective view of a portion of the torque wrench showing the first tightening socket in accordance with an exemplary embodiment.
Figure 15 is a bottom perspective view of a portion of the torque wrench showing the second tightening socket in accordance with an exemplary embodiment.
Figure 16 is a top perspective view of a portion of the torque wrench showing the second tightening socket in accordance with an exemplary embodiment.
Figure 17 is a front perspective view of a portion of the torque wrench 100 from the left side showing the loosening socket in accordance with an exemplary embodiment.
Figure 18 is a front perspective view of a portion of the torque wrench from the right side showing the loosening socket in accordance with an exemplary embodiment.
Figure 19 illustrates the torque wrench with the first tightening socket coupled to the first element and configured to be tightened by rotating the torque wrench in a tightening direction in accordance with an exemplary embodiment.
Figure 20 illustrates the torque wrench with the first tightening socket incorrectly aligned with the first element and unable to be coupled to the first element in accordance with an exemplary embodiment.
Figure 21 illustrates the torque wrench in a failed state in accordance with an exemplary embodiment.
Figure 22 illustrates the torque wrench in a failed state in accordance with an exemplary embodiment.
Figure 23 illustrates the torque wrench in a failed state in accordance with an exemplary embodiment.
Figure 24 is a top view of the torque wrench in accordance with an exemplary embodiment.
Figure 25 is a front view of the torque wrench in accordance with an exemplary embodiment.
Figure 26 is a rear view of the torque wrench in accordance with an exemplary embodiment.
Figure 27 is a bottom view of the torque wrench in accordance with an exemplary embodiment.
Figure 28 is a right side view of the torque wrench in accordance with an exemplary embodiment.
Figure 29 is a left view of the torque wrench in accordance with an exemplary embodiment.
Figure 30 shows the first element poised for loading onto the second element in accordance with an exemplary embodiment.
Figure 31 illustrates the torque wrench in accordance with an exemplary embodiment for tightening the first element to the second element.
Figure 32 illustrates the torque wrench in accordance with an exemplary embodiment for loosening the first element from the second element.
Figure 33 shows the first element being removed from the second element in accordance with an exemplary embodiment.
Figures 34 illustrates the torque wrench in accordance with an exemplary embodiment.
Figures 35 illustrates the torque wrench in accordance with an exemplary embodiment.
Figures 36 illustrates the torque wrench in accordance with an exemplary embodiment.
Figures 37 illustrates the torque wrench in accordance with an exemplary embodiment.
Figures 38 illustrates the torque wrench in accordance with an exemplary embodiment.
Figures 39 illustrates the torque wrench in accordance with an exemplary embodiment.
Figure 40 is a top perspective view of a portion of the torque wrench showing the first tightening socket in accordance with an exemplary embodiment.
Figure 41 is a bottom perspective view of a portion of the torque wrench showing the first tightening socket in accordance with an exemplary embodiment.
Figure 42 is a bottom perspective view of a portion of the torque wrench showing the second tightening socket in accordance with an exemplary embodiment.
Figure 43 is a top perspective view of a portion of the torque wrench showing the second tightening socket in accordance with an exemplary embodiment.
Figure 44 is a front perspective view of a portion of the torque wrench from the right side showing the loosening socket in accordance with an exemplary embodiment.
Figure 45 is a front perspective view of a portion of the torque wrench from the left side showing the loosening socket in accordance with an exemplary embodiment.
Figure 46 is a front perspective view of a portion of the torque wrench showing the loosening socket in accordance with an exemplary embodiment.
Figure 47 is a rear perspective view of a portion of the torque wrench showing the loosening socket in accordance with an exemplary embodiment.
Figure 48 illustrates the torque wrench with the first tightening socket coupled to the first element and configured to be tightened by rotating the torque wrench in a tightening direction in accordance with an exemplary embodiment.
Figure 49 illustrates the torque wrench with the first tightening socket incorrectly aligned with the first element and unable to be coupled to the first element in accordance with an exemplary embodiment.
Figure 50 illustrates the torque wrench in a failed state in accordance with an exemplary embodiment.
Figure 51 illustrates the torque wrench in a failed state in accordance with an exemplary embodiment.
Figure 52 illustrates the torque wrench in a failed state in accordance with an exemplary embodiment.
Figure 53 is a top view of the torque wrench in accordance with an exemplary embodiment.
Figure 54 is a front view of the torque wrench in accordance with an exemplary embodiment.
Figure 55 is a rear view of the torque wrench in accordance with an exemplary embodiment.
Figure 56 is a bottom view of the torque wrench in accordance with an exemplary embodiment.
Figure 57 is a right side view of the torque wrench in accordance with an exemplary embodiment.
Figure 58 is a left view of the torque wrench in accordance with an exemplary embodiment.
Figure 59 is a perspective view of a surgical device kit in accordance with an exemplary embodiment.
Figure 60 is a top view of the surgical device kit in accordance with an exemplary embodiment.

### DETAILED DESCRIPTION OF THE INVENTION

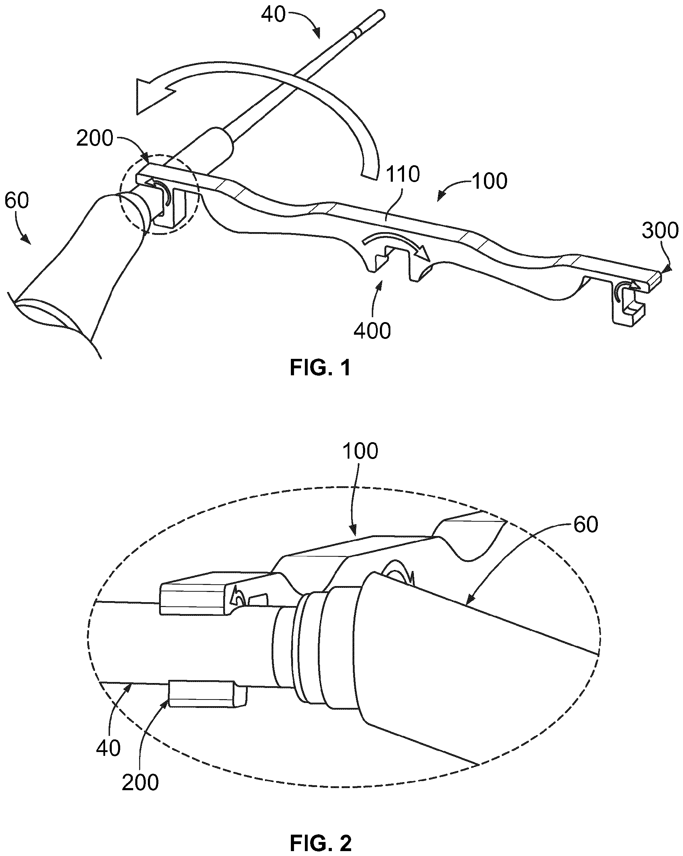

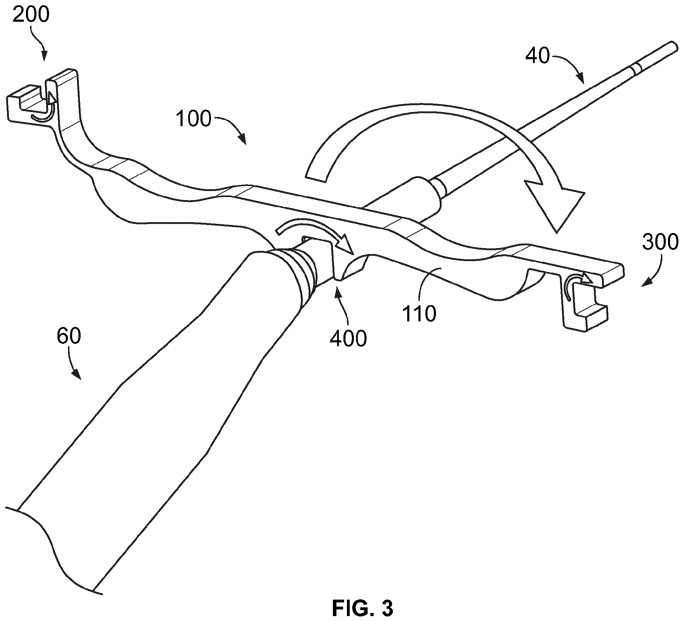

Figure 1 illustrates a torque wrench 100 in accordance with an exemplary embodiment used for tightening and loosening a first element 40 relative to a second element 60. Figure 2 is an enlarged view of the torque wrench 100 coupled to the first element 40 for tightening the first element 40 to the second element 60. Figure 3 illustrates the torque wrench 100 coupled to the first element 40 for loosening the first element 40 from the second element 60. Tightening refers to applying force to the first element 40 in a direction to connect the first element 40 to the second element 60. When tightening, the force applied to the first element 40 increases when the torque wrench 100 is rotated until the torque wrench 100 reaches the maximum torque and fails or yields. Loosening refers to applying a force to the first element 40 in a direction to disconnect the first element 40 from the second element 60. Tightening means connecting the first element 40 to the second element 60. Loosening means that the force applied to the first element to the second element decreases when the torque wrench is rotated and loosening means disconnecting the first element to the second element. In various embodiments, tightening means rotating in a clockwise direction and loosening means rotating in a counterclockwise direction, or vice versa.

In an exemplary embodiment, the torque wrench 100 is a disposable component. For example, the torque wrench 100 is configured to fail or yield at a peak torque during tightening of the first element 40 to the second element 60 to ensure that the first element 40 is properly installed on the second element 60 at a predetermined torque. In an exemplary embodiment, the torque wrench 100 is configured to deform at the predetermined torque ensuring that the first element 40 is not over tightened when installed on the second element 60. As such, the rotational orientation of the first element 40 relative to the second element 60 is achieved with high accuracy and repeatability. The torque wrench 100 is plastically deformed (for example, yields) at failure to prevent fracture of the components of the torque wrench 100 eliminating foreign objects or lost components during assembly. In an exemplary embodiment, the torque wrench 100 is manufactured from a metal material, such as an aluminum material, especially Aluminum 6061-T6, which is ductile to allow the torque wrench 100 to be plastically deformed during use. In an exemplary embodiment, the torque wrench 100 is manufactured from a material that is sterilizable. In an exemplary embodiment, the torque wrench 100 is manufactured from a material that is biocompatible. In an exemplary embodiment, the torque wrench 100 is manufactured from a material that is disposable. In an exemplary embodiment, the torque wrench 100 is manufactured from a material that is reliable. The torque wrench 100 may be used with medical or surgical instruments, such as in an operating room. In an exemplary embodiment, the torque wrench 100 is a single piece design having a unitary, monolithic structure. For example, the components used for tightening and loosening the first element 40 are part of the single unitary structure. In alternative embodiments, the torque wrench 100 may be made from multiple parts, such as having two wrench elements at opposite ends, which may be joined to each other or joined using a handle, such as a plastic handle.

In an exemplary embodiment, the torque wrench 100 includes a shaft 110 that forms the main body of the torque wrench 100. The torque wrench 100 includes one or more tightening sockets for tightening the first element 40 to the second element 60. For example, in the illustrated embodiment, the torque wrench 100 includes a first tightening socket 200 and a second tightening socket 300 at opposite ends of the shaft 110. Providing multiple tightening sockets 200, 300 allows tightening of the first element 40 on multiple occasions. For example, providing the two tightening sockets 200, 300 allows the torque wrench 100 to be a dual use disposable torque wrench. As such, if the first element 40 is incorrectly installed the first time, needs to be retorqued, or needs to be replaced with a different element, the same torque wrench 100 may be used during a subsequent tightening operation. For example, if the first element 40 needs to be removed to clean clogs or the initial installation is faulty for some reason, the torque wrench 100 may be used during the subsequent tightening operation. However, in alternative embodiments, the torque wrench 100 may have a single tightening socket 200, thus defining a single use disposable torque wrench. In an exemplary embodiment, the torque wrench 100 includes a loosening socket 400 for loosening the first element 40 from the second element 60. The loosening socket 400 is reusable to allow loosening of the first element 40 on multiple occasions. The tightening sockets 200, 300 are configured to interface with the first element 40 to only tighten the first element 40 to the second element 60 and are unable to rotate the first element 40 in the loosening direction. For example, the tightening sockets 200, 300 may include features configured to interface with the first element 40 to only rotate the first element 40 in the tightening direction, such features slipping off of the first element 40 if the torque wrench 100 is rotated in the loosening direction. Conversely, the loosening socket 400 is configured to interface with the first element 40 to only loosen the first element 40 from the second element 60 and is unable to rotate the first element 40 in the tightening direction. For example, the loosening socket 400 may include features configured to interface with the first element 40 to only rotate the first element 40 in the loosening direction, such features slipping off of the first element 40 if the torque wrench 100 is rotated in the tightening direction. Figures 1 and 2 illustrates the first tightening socket 200 coupled to the first element 40 to perform a tightening operation by rotating the torque wrench 100 in a tightening direction (for example, counterclockwise). The first tightening socket 200 is configured to interface with the first element 40 to only tighten the first element 40 to the second element 60 and is unable to rotate the first element 40 in the loosening direction. Figure 3 illustrates the loosening socket 400 coupled to the first element 40 to perform a loosening operation by rotating the torque wrench 100 in a loosening direction (for example, clockwise). Figure 3 illustrates the first tightening socket 200 deformed and in a failed or yielded state. However, Figure 3 illustrates the second tightening socket 300 in a normal, i.e. proper-working and efficient, state, which allows for a second tightening operation by the torque wrench 100 prior to disposal of the torque wrench 100.

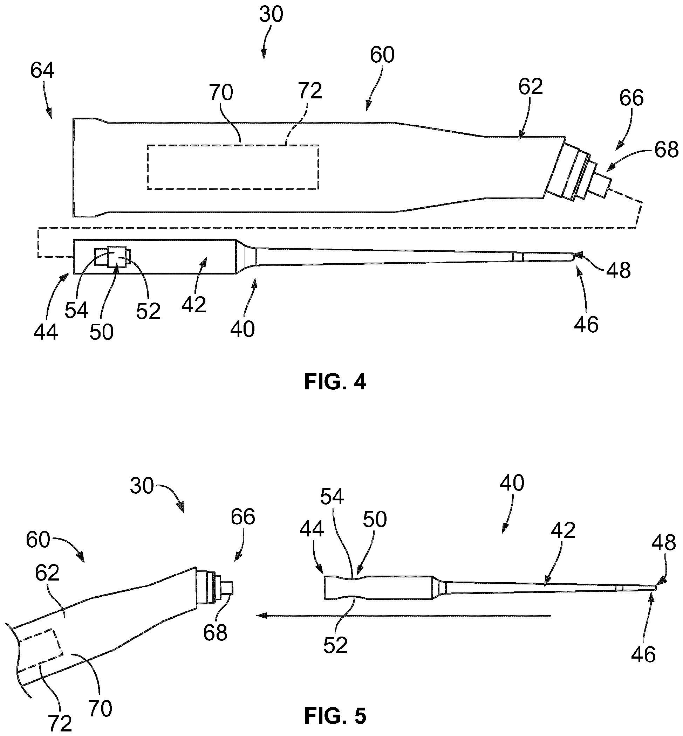

With additional reference to Figures 4 and 5, exemplary embodiments of the first and second elements 40, 60 are shown. Figure 4 is an exploded view of a surgical device 30 that may be assembled using the first and second elements 40, 60. Figure 5 shows the first element 40 poised for coupling to the second element 60. In the illustrated embodiment, the first element 40 is a surgical attachment 42 and the second element 60 is a handpiece 62. In an exemplary embodiment, the handpiece 62 is an ultrasonic handpiece configured to generate ultrasonic motion of the surgical attachment 42. The surgical attachment 42 is a working element usable during a surgical procedure. For example, the surgical attachment 42 may be used to remove soft tissue and/or fibrous tissue and/or hard tissue from a patient during a surgical procedure.

In various embodiments, the first element 40 is cylindrical (for example, has a circular cross-section) and/or includes multiple cylindrical portions along the length of the first element 40. Portions of the first element 40 may be stepped having different diameters. Portions of the first element 40 may be tapered. The first element 40 may have other shapes in alternative embodiments, such as a rectangular cross-section.

The first element 40 extends between a proximal end 44 and a distal end 46. The proximal end 44 is configured to be coupled to the second element 60. For example, the proximal end 44 may be threadably coupled to the second element 60. The proximal end 44 may include a threaded bore or a threaded post for threadably coupling to the second element 60. The torque wrench 100 is used to threadably couple the proximal end 44 of the first element 40 to the second element 60. In an exemplary embodiment, the first element 40 includes a working element 48 at the distal end 46. The working element 48 is configured to perform an action, such as interfacing with the patient tissue during the surgical procedure. The working element 48 may include a cutting blade, cutting teeth, an aspiration port, an irrigation port, and/or other surgical elements for performing a surgical procedure.

In an exemplary embodiment, the first element 40 includes a torque wrench interface 50 along the exterior of the first element 40. The torque wrench 100 is configured to engage the first element 40 at the torque wrench interface 50. In an exemplary embodiment, the first element 40 includes one or more drive elements 52 at the torque wrench interface 50. The torque wrench 100 engages the drive elements 52 to rotate the first element 40 during the tightening or loosening operations. In an exemplary embodiment, the drive elements 52 include one or more flat surfaces 54 along the exterior of the first element 40. The torque wrench 100 is configured to engage the first element 40 at the flat surfaces 54 to rotate the first element 40. In various embodiments, the flat surfaces 54 may be provided on opposite sides of the first element 40 (for example, along the top and bottom of the first element 40). In other various embodiments, the first element 40 may be hexagonal shaped at the torque wrench interface 50 having six of the flat surfaces 54 arranged on six sides of the first element 40.

The second element 60 extends between a proximal end 64 and a distal end 66. The first element 40 is configured to be coupled to the distal end 66. For example, the first element 40 may be rotatably coupled to the distal end 66. The distal end 66 may include a threaded post or a threaded bore configured to be threadably coupled to the first element 40. In the illustrated embodiment, the second element 60 includes a threaded adapter 68 at the distal end 66. The first element 40 is configured be threadably coupled to the threaded adapter 68. The torque wrench 100 is used to tighten the first element 40 onto the threaded adapter 68 and loosen the first element 40 from the threaded adapter 68. The torque wrench 100 is configured to tighten the first element 40 to a predetermined torque to ensure proper attachment of the first element 40 to the second element 60.

In an exemplary embodiment, the second element 60 includes a housing 70 holding an ultrasonic transducer 72 that generates ultrasonic motion of the first element 40 attached to the end of the second element 60. In an exemplary embodiment, the ultrasonic transducer 72 generates torsional motion of the first element 40 along a central axis of the first element 40. The ultrasonic transducer 72 may additionally or alternatively generate longitudinal motion of the first element 40 along the central axis of the first element 40. In an exemplary embodiment, the second element 60 includes an aspiration port and/or in the irrigation port to provide aspiration and/or irrigation at the surgical site.

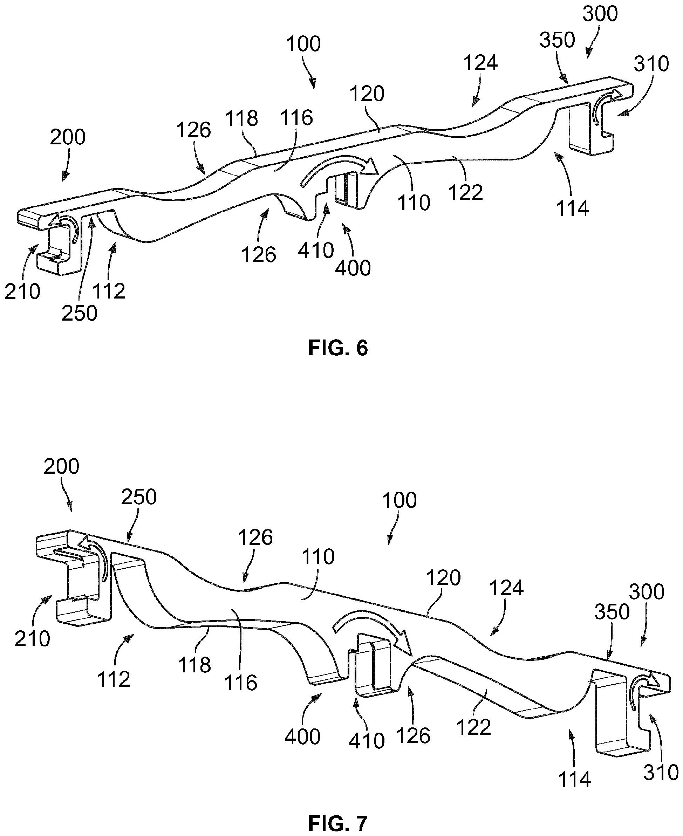

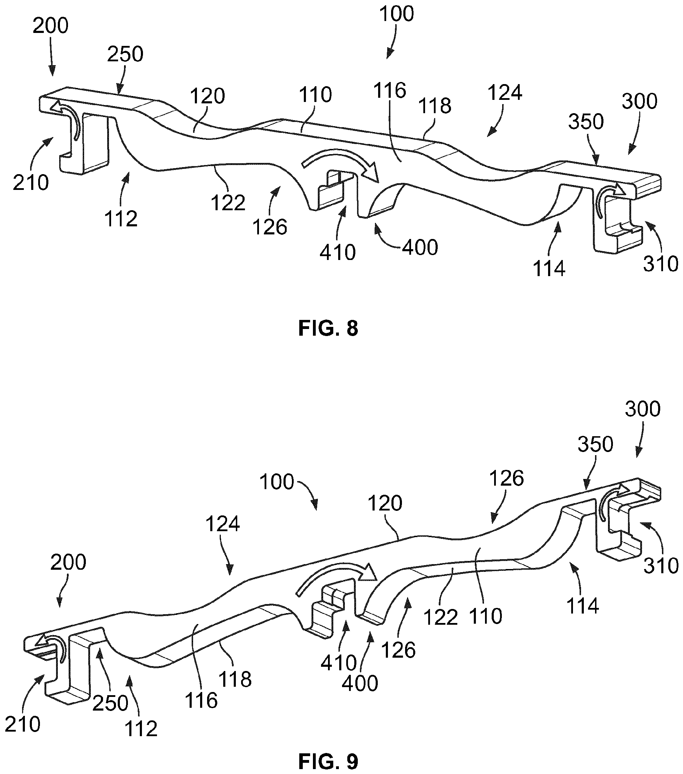

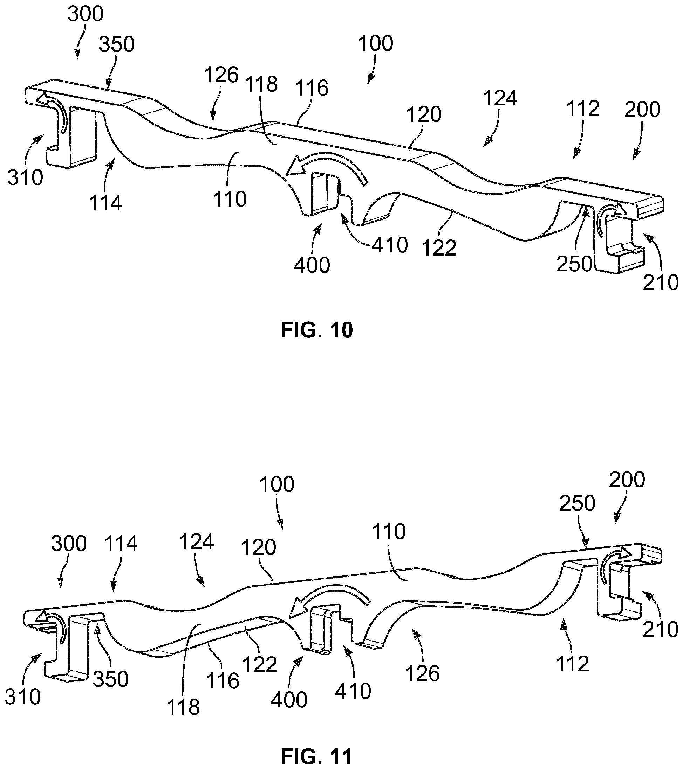

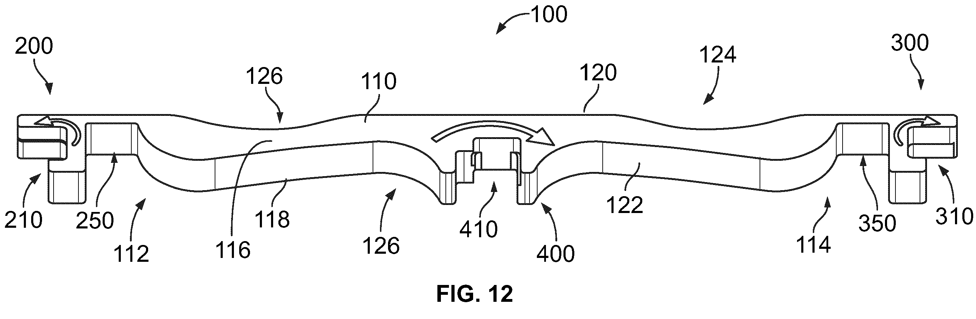

Figures 6 through 12 illustrate the torque wrench 100 in accordance with an exemplary embodiment. Figure 6 is a top perspective, front, right side view of the torque wrench 100. Figure 7 is a bottom perspective, front, right side view of the torque wrench 100. Figure 8 is a top perspective, front, left side view of the torque wrench 100. Figure 9 is a bottom perspective, front, left side view of the torque wrench 100. Figure 10 is a top perspective, rear, right side view of the torque wrench 100. Figure 11 is a bottom perspective, rear, right side view of the torque wrench 100. Figure 12 is a bottom perspective, front view of the torque wrench 100.

The torque wrench 100 includes the shaft 110, the first tightening socket 200, the second tightening socket 300, and the loosening socket 400. In an alternative embodiment, the torque wrench 100 may be provided without the second tightening socket 300 and/or the loosening socket 400. In an exemplary embodiment, the torque wrench 100 is formed from a subtractive manufacturing process, such as using a CNC machine, manual machining, milling, cutting, or otherwise machining the part. In various embodiments, the torque wrench 100 is stamped and formed from a sheet of metal, such as an aluminum sheet. In other various embodiments, the torque wrench 100 may be diecast from a metal material. In other various embodiments, the torque wrench 100 may be extruded. In other embodiments, the torque wrench 100 may be additive manufactured. The torque wrench 100 may be lightweight and easy to hold. For example, the torque wrench 100 may have ergonomic features that allow the torque wrench 100 to be easily handled by the operator to tighten and loosen the first element 40.

The shaft 110 extends between a first end 112 at the right side and a second end 114 at the left side. The shaft 110 includes a front 116 and a rear 118. The shaft 110 includes a top 120 and a bottom 122. In an exemplary embodiment, the first tightening socket 200 is provided at the first end 112 and the second tightening socket 300 is provided at the second end 114. Other locations are possible in alternative embodiments. In an exemplary embodiment, the loosening socket 400 is provided at the bottom 122. In the illustrated embodiment, the loosening socket 400 is approximately centered between the first end 112 and the second end 114. Other locations are possible in alternative embodiments. In an exemplary embodiment, the front 116 and/or the rear 118 are planar. The front 116 may be parallel to the rear 118.

In an exemplary embodiment, the shaft 110 includes a handle 124 that is used to grip the torque wrench 100 during tightening and/or loosening. The handle 124 may be defined by gripping features 126 along the shaft 110. For example, the gripping features 126 may be defined by curved surfaces along the top 120 and/or the bottom 122 providing areas for the operators fingers to ergonomically and securely hold the shaft 110 during the tightening and/or loosening operations.

The first tightening socket 200 includes a first spanner pocket 210 configured to receive the first element 40 to tighten the first element 40 when the torque wrench 100 is rotated in a tightening direction. In the illustrated embodiment, the first spanner pocket 210 is open-ended to easily receive and release from the first element 40. However, the first spanner pocket 210 may be closed (for example, in closing or circumferentially surrounding the pocket) in alternative embodiments. The first tightening socket 200 is connected to the first end 112 of the shaft 110 by a first weakened section 250 configured to yield at the predetermined torque. The first weakened section 250 is located between the first spanner pocket 210 and the first end 112 of the shaft 110.

The second tightening socket 300 includes a second spanner pocket 310 configured to receive the first element 40 to tighten the first element 40 when the torque wrench 100 is rotated in a tightening direction. In the illustrated embodiment, the second spanner pocket 310 is open-ended to easily receive and release from the first element 40. However, the second spanner pocket 310 may be closed (for example, in closing or circumferentially surrounding the pocket) in alternative embodiments. The second tightening socket 300 is connected to the second end 114 of the shaft 110 by a second weakened section 350 configured to yield at the predetermined torque. The second weakened section 350 is located between the second spanner pocket 310 and the second end 114 of the shaft 110.

The loosening socket 400 is separate and discrete from the first tightening socket 200 and the second tightening socket 300. For example, the loosening socket 400 is located remote from the first tightening socket 200 and from the second tightening socket 300. The loosening socket 400 is configured to receive the first element 40 during a loosening operation. The loosening socket 400 includes a loosening spanner pocket 410 configured to receive the first element 40 to loosen the first element 40 from the second element 60 when the torque wrench 100 is rotated in a loosening direction.

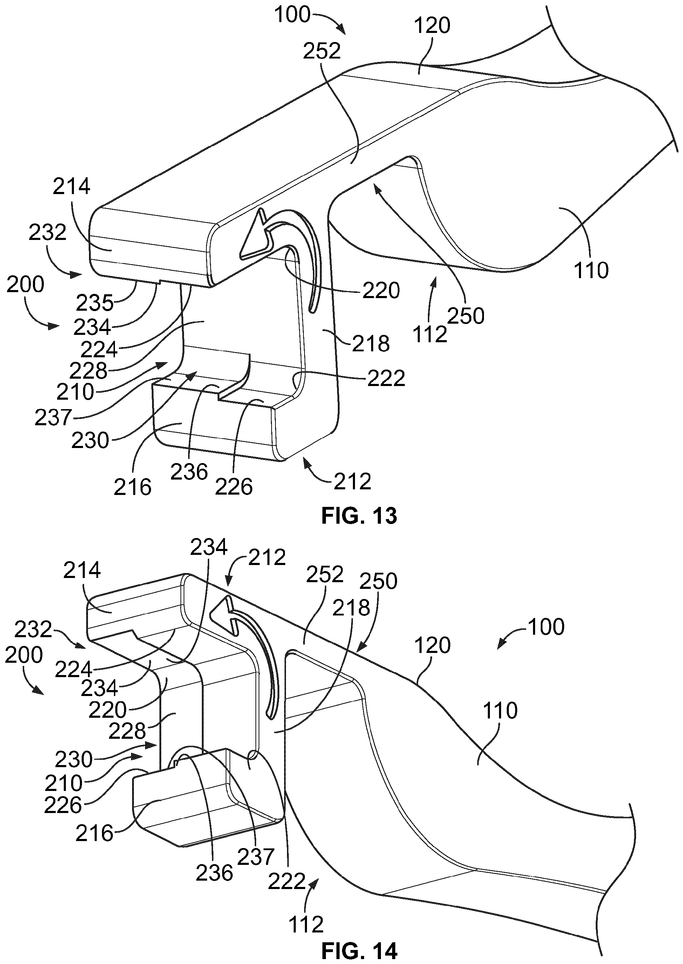

Figure 13 is a top perspective view of a portion of the torque wrench 100 showing the first tightening socket 200 in accordance with an exemplary embodiment. Figure 14 is a bottom perspective view of a portion of the torque wrench 100 showing the first tightening socket 200 in accordance with an exemplary embodiment. The first tightening socket 200 includes the first spanner pocket 210 and the first weakened section 250 connecting the first spanner pocket 210 to the first end 112 of the shaft 110.

In an exemplary embodiment, the first tightening socket 200 includes a first head 212 forming the first spanner pocket 210. The first head 212 includes an upper jaw 214, a lower jaw 216, and a spanner wall 218 between the upper jaw 214 and the lower jaw 216. The first spanner pocket 210 is bounded by the upper jaw 214, the lower jaw 216, and the spanner wall 218. In an exemplary embodiment, the first spanner pocket 210 is open between the upper jaw 214 and the lower jaw 216 opposite the spanner wall 218 to receive the first element 40. For example, the first element 40 may be side loaded into the first spanner pocket 210 between the upper jaw 214 and the lower jaw 216. In the illustrated embodiment, the lower jaw 216 extends parallel to the upper jaw 214. In the illustrated embodiment, the spanner wall 218 extends perpendicular to the upper jaw 214 and/or the lower jaw 216. The first head 212 may have other shapes in alternative embodiments.

In various embodiments, the upper jaw 214 is longer than the lower jaw 216 (the lower jaw 216 is shorter than the upper jaw 214), which provides a feature to ensure that the first tightening socket 200 only tightens the first element 40 in a single direction. For example, if the torque wrench 100 is rotated in the wrong direction (for example, the loosening direction), the shortened lower jaw 216 causes the first tightening socket 200 to immediately separate from the first element 40 rather than rotating the first element 40 in the loosening direction.

In an exemplary embodiment, the spanner wall 218 meets the upper jaw 214 at an upper corner 220 and the spanner wall 218 meets the lower jaw 216 at a lower corner 222. The corners 220, 222 may be approximately 90° corners. Optionally, the corners 220, 222 may be curved. In an exemplary embodiment, the upper jaw 214, the lower jaw 216, and the spanner wall 218 include interface surfaces 224, 226, 228, respectively, at the interior surfaces that form a receiving pocket 230 for receiving the first element 40. In an exemplary embodiment, the interface surfaces 224, 226, 228 include flat surfaces configured to interface with the first element 40 to rotate the first element 40 in the tightening direction.

In an exemplary embodiment, the first weakened section 250 ties into the upper jaw 214. For example, the upper jaw 214 is co-planer with the first weakened section 250. The upper jaw 214 may be generally aligned with the top 120 of the shaft 110. For example, the upper jaw 214 may be co-planer with the top 120 of the shaft 110. In alternative embodiments, the upper jaw 214 may be located above the top 120 of the shaft 110. For example, the first weakened section 250 may tie into the spanner wall 218 or the lower jaw 216. In other various embodiments, the upper jaw 214 may be angled transverse relative to the top 120 of the shaft 110. For example, the first head 212 may be angled downward with the upper jaw 214 and/or the first weakened section 250 oriented at a downward angle relative to the top 120 of the shaft 110. Alternatively, the first head 212 may be angled upward with the upper jaw 214 and/or the first weakened section 250 oriented at an upward angle relative to the top 120 of the shaft 110.

In an exemplary embodiment, the first tightening socket 200 includes one or more keying features 232 for keyed mating with the first element 40. The keyed mating with the first element 40 ensures that the first tightening socket 200 is mated with the first element 40 in only one orientation to ensure that the first element 40 is rotated in the correct direction when the torque wrench 100 is used to tighten the first element 40. In an exemplary embodiment, the keying features 232 include an upper keying feature 234 on the upper jaw 214 and a lower keying feature 236 on the lower jaw 216. In alternative embodiments, the upper keying feature 234 or the lower keying feature 236 may be eliminated. The keying features 232 may be provided at other locations in alternative embodiments. In an exemplary embodiment, the upper keying feature 234 includes a platform 235 stepped inward relative to the interface surface 224 of the upper jaw 214. The platform 235 may have a flat surface configured to interface with the first element 40 to rotate the first element 40 during the tightening process. In the illustrated embodiment, the platform 235 is located toward the rear of the first tightening socket 200. Other locations are possible in alternative embodiments, such as at the front or approximately centered on the upper jaw 214. In the illustrated embodiment, the platform 235 has a width approximately half of a width of the upper jaw 214. However, the platform 235 may be wider or narrower in alternative embodiments. In an exemplary embodiment, the lower keying feature 236 includes a platform 237 stepped inward relative to the interface surface 226 of the lower jaw 216. The platform 237 may have a flat surface configured to interface with the first element 40 to rotate the first element 40 during the tightening process. In the illustrated embodiment, the platform 237 is located toward the rear of the first tightening socket 200. Other locations are possible in alternative embodiments, such as at the front or approximately centered on the lower jaw 216. In the illustrated embodiment, the platform 237 has a width approximately half of the width of the lower jaw 216. However, the platform 237 may be wider or narrower in alternative embodiments. In an exemplary embodiment, the keying features 234, 236 are arranged (for example, both at the rear) to prevent the torque wrench 100 from being coupled to the first element 40 in an incorrect orientation (for example, upside down). The keying features 232 may be optional elements. For example, the torque wrench 100 may be provided without the keying features 232 in alternative embodiments.

The first weakened section 250 extends between the first spanner pocket 210 and the first end 112 of the shaft 110. The first weakened section 250 includes a first neck 252 located between the shaft 110 and the first head 212. The first neck 252 is located remote from the receiving pocket 230 of the first spanner pocket 210 such that the first weakened section 250 may be spaced apart from the first element 40. As such, the engaging elements of the first tightening socket 200 remain intact and do not deform during use of the torque wrench 100. Rather, the deformation occurs remote from the first element 40. In the illustrated embodiment, the first neck 252 is planar or flat in the normal state prior to deformation. However, the first neck 252 may be angled or bent downward or angled or bent upward in the normal state prior to deformation. In the illustrated embodiment, the neck 252 is located at the top of the torque wrench 100. For example, the neck 252 may be aligned with the top 120 of the shaft 110. The neck 252 may be aligned with the upper jaw 214. In alternative embodiments, the neck 252 may be tied into the spanner wall 218, such as at a location between the upper jaw 214 and the lower jaw 216.

The neck 252 of the first weakened section 250 has a reduced cross-sectional area compared to the shaft 110. For example, the first weakened section 250 has a reduced height compared to a height of the shaft 110. The first weakened section 250 may additionally or alternatively have a reduced width compared to a width of the shaft 110. The neck 252 is weakened compared to the shaft 110 to define the area of deformation when the torque limit of the first tightening socket 200 is reached. For example, when the predetermined peak torque is reached during the tightening process, the first weakened section 250 starts to bend and deform rather than transfer the rotation of the shaft 110 to the first element 40. The torque wrench 100 is manufactured from a ductile material, such as an aluminum material, allowing for plastic deformation when the predetermined torque limit is reached. The first weakened section 250 may consist of a material with a smaller stiffness compared to the material of the shaft 110.

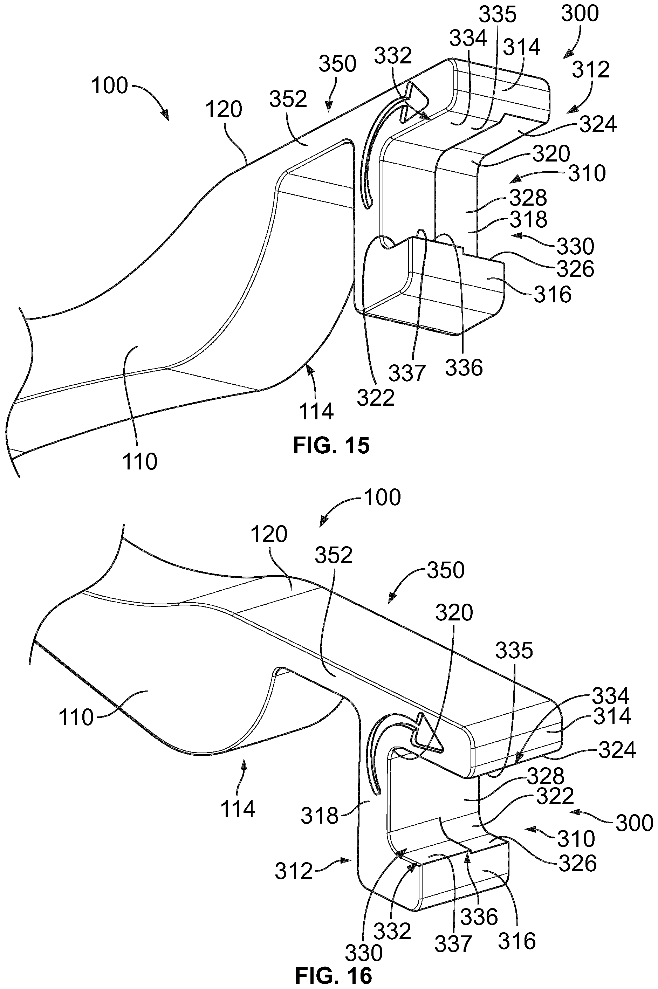

Figure 15 is a bottom perspective view of a portion of the torque wrench 100 showing the second tightening socket 300 in accordance with an exemplary embodiment. Figure 16 is a top perspective view of a portion of the torque wrench 100 showing the second tightening socket 300 in accordance with an exemplary embodiment. The second tightening socket 300 includes the second spanner pocket 310 and the second weakened section 350 connecting the second spanner pocket 310 to the second end 114 of the shaft 110. In various embodiments, the second tightening socket 300 may be identical to the first tightening socket 300, simply being arranged at the opposite end of the torque wrench 100. However, the second tightening socket 300 may be different in alternative embodiment, such as for use on a different size element or for torquing to a different peak torque. For example, different elements (for example, different surgical attachments) may be configured to be attached to the second element (for example, the handpiece), which may require a different sized or shaped pocket and/or may require a different torque amount for proper installation.

In an exemplary embodiment, the second tightening socket 300 includes a second head 312 forming the second spanner pocket 310. The second head 312 includes an upper jaw 314, a lower jaw 316, and a spanner wall 318 between the upper jaw 314 and the lower jaw 316. The second spanner pocket 310 is bounded by the upper jaw 314, the lower jaw 316, and the spanner wall 318. In an exemplary embodiment, the second spanner pocket 310 is open between the upper jaw 314 and the lower jaw 316 opposite the spanner wall 318 to receive the first element 40. For example, the first element 40 may be side loaded into the second spanner pocket 310 between the upper jaw 314 and the lower jaw 316. In the illustrated embodiment, the lower jaw 316 extends parallel to the upper jaw 314. In the illustrated embodiment, the spanner wall 318 extends perpendicular to the upper jaw 314 and/or the lower jaw 316. The second head 312 may have other shapes in alternative embodiments.

In various embodiments, the upper jaw 314 is longer than the lower jaw 316 (the lower jaw 316 is shorter than the upper jaw 314), which provides a feature to ensure that the second tightening socket 300 only tightens the first element 40 in a single direction. For example, if the torque wrench 100 is rotated in the wrong direction (for example, the loosening direction), the shortened lower jaw 316 causes the second tightening socket 300 to immediately separate from the first element 40 rather than rotating the first element 40 in the loosening direction.

In an exemplary embodiment, the spanner wall 318 meets the upper jaw 314 at an upper corner 320 and the spanner wall 318 meets the lower jaw 316 at a lower corner 322. The corners 320, 322 may be approximately 90° corners. Optionally, the corners 320, 322 may be curved. In an exemplary embodiment, the upper jaw 314, the lower jaw 316, and the spanner wall 318 include interface surfaces 324, 326, 328, respectively, at the interior surfaces that form a receiving pocket 330 for receiving the first element 40. In an exemplary embodiment, the interface surfaces 324, 326, 328 include flat surfaces configured to interface with the first element 40 to rotate the first element 40 in the tightening direction.

In an exemplary embodiment, the second weakened section 350 ties into the upper jaw 314. For example, the upper jaw 314 is co-planer with the second weakened section 350. The upper jaw 314 may be generally aligned with the top 120 of the shaft 110. For example, the upper jaw 314 may be co-planer with the top 120 of the shaft 110. In alternative embodiments, the upper jaw 314 may be located above the top 120 of the shaft 110. For example, the second weakened section 350 may tie into the spanner wall 318 or the lower jaw 316. In other various embodiments, the upper jaw 314 may be angled transverse relative to the top 120 of the shaft 110. For example, the second head 312 may be angled downward with the upper jaw 314 and/or the second weakened section 350 oriented at a downward angle relative to the top 120 of the shaft 110. Alternatively, the second head 312 may be angled upward with the upper jaw 314 and/or the second weakened section 350 oriented at an upward angle relative to the top 120 of the shaft 110.

In an exemplary embodiment, the second tightening socket 300 includes one or more keying features 332 for keyed mating with the first element 40. The keyed mating with the first element 40 ensures that the second tightening socket 300 is mated with the first element 40 in only one orientation to ensure that the first element 40 is rotated in the correct direction when the torque wrench 100 is used to tighten the first element 40. In an exemplary embodiment, the keying features 332 include an upper keying feature 334 on the upper jaw 314 and a lower keying feature 336 on the lower jaw 316. In alternative embodiments, the upper keying feature 334 or the lower keying feature 336 may be eliminated. The keying features 332 may be provided at other locations in alternative embodiments. In an exemplary embodiment, the upper keying feature 334 includes a platform 335 stepped inward relative to the interface surface 324 of the upper jaw 314. The platform 335 may have a flat surface configured to interface with the first element 40 to rotate the first element 40 during the tightening process. In the illustrated embodiment, the platform 335 is located toward the rear of the second tightening socket 300. Other locations are possible in alternative embodiments, such as at the front or approximately centered on the upper jaw 314. In the illustrated embodiment, the platform 335 has a width approximately half of a width of the upper jaw 314. However, the platform 335 may be wider or narrower in alternative embodiments. In an exemplary embodiment, the lower keying feature 336 includes a platform 337 stepped inward relative to the interface surface 326 of the lower jaw 316. The platform 337 may have a flat surface configured to interface with the second element 40 to rotate the first element 40 during the tightening process. In the illustrated embodiment, the platform 337 is located toward the rear of the second tightening socket 300. Other locations are possible in alternative embodiments, such as at the front or approximately centered on the lower jaw 316. In the illustrated embodiment, the platform 337 has a width approximately half of the width of the lower jaw 316. However, the platform 337 may be wider or narrower in alternative embodiments. In an exemplary embodiment, the keying features 334, 336 are arranged (for example, both at the rear) to prevent the torque wrench 100 from being coupled to the first element 40 in an incorrect orientation (for example, upside down). The keying features 332 may be optional elements. For example, the torque wrench 100 may be provided without the keying features 232 in alternative embodiments.

The second weakened section 350 extends between the second spanner pocket 310 and the second end 114 of the shaft 110. The second weakened section 350 includes a second neck 352 located between the shaft 110 and the second head 312. The second neck 352 is located remote from the receiving pocket 330 of the second spanner pocket 310 such that the second weakened section 350 may be spaced apart from the first element 40. As such, the engaging elements of the second tightening socket 300 remain intact and do not deform during use of the torque wrench 100. Rather, the deformation occurs remote from the first element 40. In the illustrated embodiment, the second neck 352 is planar or flat in the normal state prior to deformation. However, the second neck 352 may be angled or bent downward or angled or bent upward in the normal state prior to deformation. In the illustrated embodiment, the neck 352 is located at the top of the torque wrench 100. For example, the neck 352 may be aligned with the top 120 of the shaft 110. The neck 352 may be aligned with the upper jaw 314. In alternative embodiments, the neck 352 may be tied into the spanner wall 318, such as at a location between the upper jaw 314 and the lower jaw 316.

The neck 352 of the second weakened section 350 has a reduced cross-sectional area compared to the shaft 110. For example, the second weakened section 350 has a reduced height compared to a height of the shaft 110. The second weakened section 350 may additionally or alternatively have a reduced width compared to a width of the shaft 110. The reduced cross-sectional area of the second weakened section 350 may be the same as the reduced cross-sectional area of the first weakened section 250 leading to both weakened sections 350 failing at the same peak torque. However, if the weakened sections 250, 350 have different cross-sectional areas, the weakened sections 250, 350 may yield at different peak torques. The neck 352 is weakened compared to the shaft 110 to define the area of deformation when the torque limit of the second tightening socket 300 is reached. For example, when the predetermined peak torque is reached during the tightening process, the second weakened section 350 starts to bend and deform rather than transfer the rotation of the shaft 110 to the first element 40. The torque wrench 100 is manufactured from a ductile material, such as an aluminum material, allowing for plastic deformation when the predetermined torque limit is reached. The second weakened section 350 may consist of a material with a smaller stiffness compared to the material of the shaft 110.

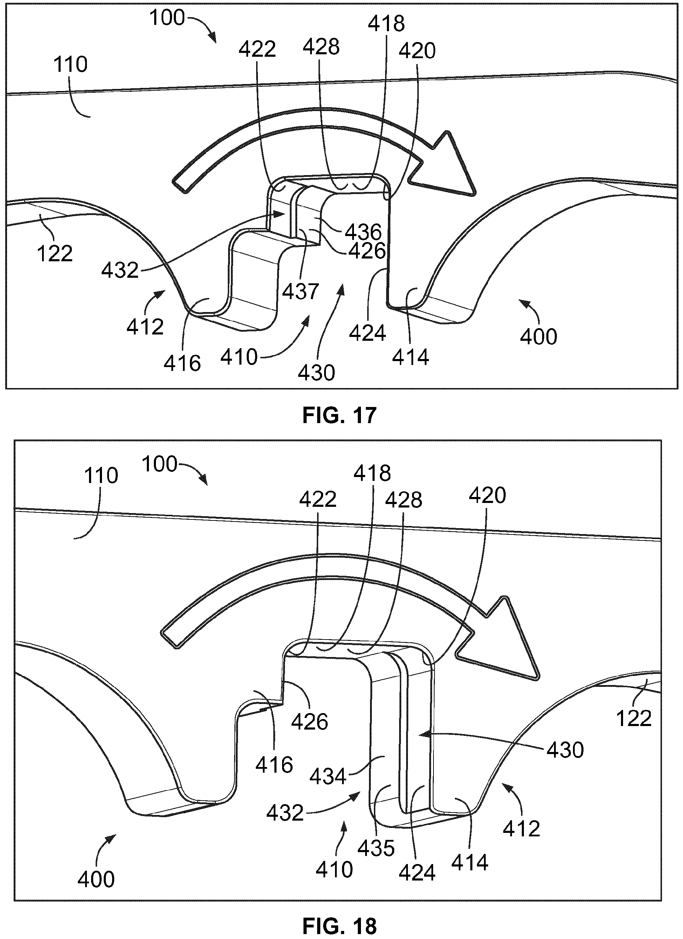

Figure 17 is a front perspective view of a portion of the torque wrench 100 from the left side showing the loosening socket 400 in accordance with an exemplary embodiment. Figure 18 is a front perspective view of a portion of the torque wrench 100 from the right side showing the loosening socket 400 in accordance with an exemplary embodiment. The loosening socket 400 includes the loosening spanner pocket 410 at the bottom 122 of the shaft 110.

In an exemplary embodiment, the loosening socket 400 includes a loosening head 412 forming the loosening spanner pocket 410. The loosening head 412 includes a first jaw 414, a second jaw 416, and a spanner wall 418 between the first jaw 414 and the second jaw 416. The loosening spanner pocket 410 is bounded by the first jaw 414, the second jaw 416, and the spanner wall 418. In an exemplary embodiment, the loosening spanner pocket 410 is open between the first jaw 414 and the second jaw 416 opposite the spanner wall 418 to receive the first element 40. For example, the first element 40 may be loaded into the loosening spanner pocket 410 through the bottom between the first jaw 414 and the second jaw 416. In the illustrated embodiment, the second jaw 416 extends parallel to the first jaw 414. In the illustrated embodiment, the spanner wall 418 extends perpendicular to the first jaw 414 and/or the second jaw 416. The loosening head 412 may have other shapes in alternative embodiments.

In various embodiments, the first jaw 414 is longer than the second jaw 416 (the second jaw 416 is shorter than the first jaw 414), which provides a feature to ensure that the loosening socket 400 only loosens the first element 40 in a single direction. For example, if the torque wrench 100 is rotated in the wrong direction (for example, the tightening direction), the shortened second jaw 416 causes the loosening socket 400 to immediately separate from the first element 40 rather than rotating the first element 40 in the tightening direction.

In an exemplary embodiment, the spanner wall 418 meets the first jaw 414 at a first corner 420 and the spanner wall 418 meets the second jaw 416 at a second corner 422. The corners 420, 422 may be approximately 90° corners. Optionally, the corners 420, 422 may be curved. In an exemplary embodiment, the first jaw 414, the second jaw 416, and the spanner wall 418 include interface surfaces 424, 426, 428, respectively, at the interior surfaces that form a receiving pocket 430 for receiving the first element 40. In an exemplary embodiment, the interface surfaces 424, 426, 428 include flat surfaces configured to interface with the first element 40 to rotate the first element 40 in the loosening direction.

In an exemplary embodiment, the loosening socket 400 includes one or more keying features 432 for keyed mating with the first element 40. The keyed mating with the first element 40 ensures that the loosening socket 400 is mated with the first element 40 in only one orientation to ensure that the first element 40 is rotated in the correct direction when the torque wrench 100 is used to tighten the first element 40. In an exemplary embodiment, the keying features 432 include a first keying feature 434 on the first jaw 414 and a second keying feature 436 on the second jaw 416. In alternative embodiments, the first keying feature 434 or the second keying feature 436 may be eliminated. The keying features 432 may be provided at other locations in alternative embodiments. In an exemplary embodiment, the first keying feature 434 includes a platform 435 stepped inward relative to the interface surface 424 of the first jaw 414. The platform 435 may have a flat surface configured to interface with the first element 40 to rotate the first element 40 during the tightening process. In the illustrated embodiment, the platform 435 is located toward the rear of the loosening socket 400. Other locations are possible in alternative embodiments, such as at the front or approximately centered on the first jaw 414. In the illustrated embodiment, the platform 435 has a width approximately half of a width of the first jaw 414. However, the platform 435 may be wider or narrower in alternative embodiments. In an exemplary embodiment, the second keying feature 436 includes a platform 437 stepped inward relative to the interface surface 426 of the second jaw 416. The platform 437 may have a flat surface configured to interface with the second element 40 to rotate the first element 40 during the tightening process. In the illustrated embodiment, the platform 437 is located toward the rear of the loosening socket 400. Other locations are possible in alternative embodiments, such as at the front or approximately centered on the second jaw 416. In the illustrated embodiment, the platform 437 has a width approximately half of the width of the second jaw 416. However, the platform 437 may be wider or narrower in alternative embodiments. In an exemplary embodiment, the keying features 434, 436 are arranged (for example, both at the rear) to prevent the torque wrench 100 from being coupled to the first element 40 in an incorrect orientation (for example, upside down).

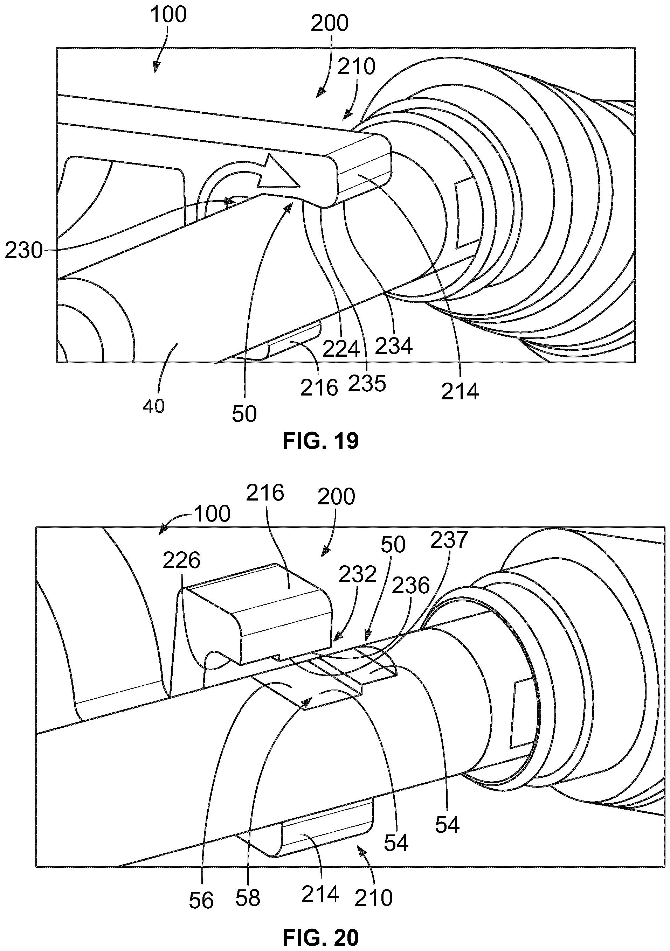

Figure 19 illustrates the torque wrench 100 with the first tightening socket 200 coupled to the first element 40 and configured to be tightened by rotating the torque wrench 100 in a tightening direction. Figure 20 illustrates the torque wrench 100 with the first tightening socket 200 incorrectly aligned with the first element 40 and unable to be coupled to the first element 40. Figure 20 shows the torque wrench 100 inverted 180° (for example, upside down) such that the first tightening socket 200 is unable to receive the first element 40. For example, the keying features 232 prevent the first tightening socket 200 from coupling to the first element 40 in an incorrect orientation.

In an exemplary embodiment, the first element 40 includes keying features 56 at the torque wrench interface 50. When the torque wrench 100 is properly oriented relative to the first element 40, the interface surfaces 224, 226 of the upper and lower jaws 214, 216 are aligned with the flat surfaces 54 at the torque wrench interface 50. The interface surfaces 234, 236 are configured to seat on the flat surfaces 54 and are used to drive the first element 40 in the tightening direction when the torque wrench 100 is rotated in the tightening direction. The keying features 234, 236 on the upper and lower jaws 214, 216 are configured to interface with the keying features 56 of the first element 40. In the illustrated embodiment, the keying feature 56 include notches 58 that are stepped inward to receive the platforms 235, 237 defining the keying features 234, 236. The platforms and the notches may be reversed in alternative embodiments (for example with the platforms on the first element 40 and the notches on the jaws of the tightening socket). Other types of keying features may be used in alternative embodiments, such as slots, grooves, tabs, protrusions, and the like. When the torque wrench 100 is improperly oriented (Figure 20), the first spanner pocket 210 is unable to receive the first element 40. The keying features 234, 236 are offset or misaligned relative to the keying features 56 to prevent loading of the first element 40 into the receiving pocket 230.

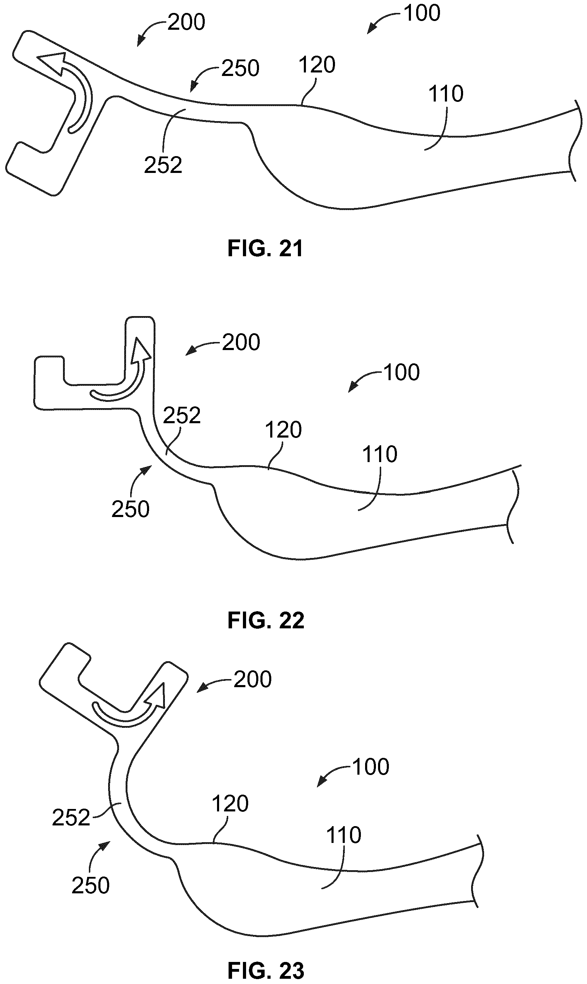

Figure 21 illustrates the torque wrench 100 in a failed (or yielded) state in accordance with an exemplary embodiment. Figure 22 illustrates the torque wrench 100 in a failed state in accordance with an exemplary embodiment. Figure 23 illustrates the torque wrench 100 in a failed state in accordance with an exemplary embodiment. Figures 21-23 shows the first weakened section 250 deformed. For example, the neck 252 is bent upward and is no longer planar. The first weakened section 250 is deformed during the tightening process when the torque limit is reached.

Figure 21 illustrates the first weakened section 250 deflected approximately 15°. Figure 22 illustrates the first weakened section 250 deflected approximately 90°. Figure 23 illustrates the first weakened section 250 deflected approximately 120°. The amount of deformation depends on the amount of rotation of the torque wrench 100 in the tightening direction after the peak torque is reached. The amount of deformation may be in the range of between approximately 0° and 180° while maintaining a consistent torque through deformation that is no greater than the intended peak torque. However, the amount of deformation may depend on the starting orientation of the first tightening socket 200 relative to the shaft 110. The amount of deformation may be limited by the shaft 110. For example, the first tightening socket 200 may be deformed until the first tightening socket 200 bottoms out against the top 120 of the shaft 110. Optionally, the first tightening socket 200 may have multiple uses. For example, the first tightening socket 200 may be used during a first tightening operation and deformed to the state shown in Figure 21, then may be used during a second tightening operation and deformed to the state shown in Figure 22, then may be used during a third tightening operation and deformed to the state shown in Figure 23. For example, the first weekend section 250 has a consistent peak torque regardless of the starting orientation. For example, the first weakened section 250 deformed at the same peak torque from the normal state to the state shown in Figure 21 as compared to from the state shown in Figure 21 to the state shown in Figure 22 as compared to from the state shown in Figure 22 to the state shown in Figure 23. The torque wrench 100 has a high degree of accuracy and consistency of deformation.

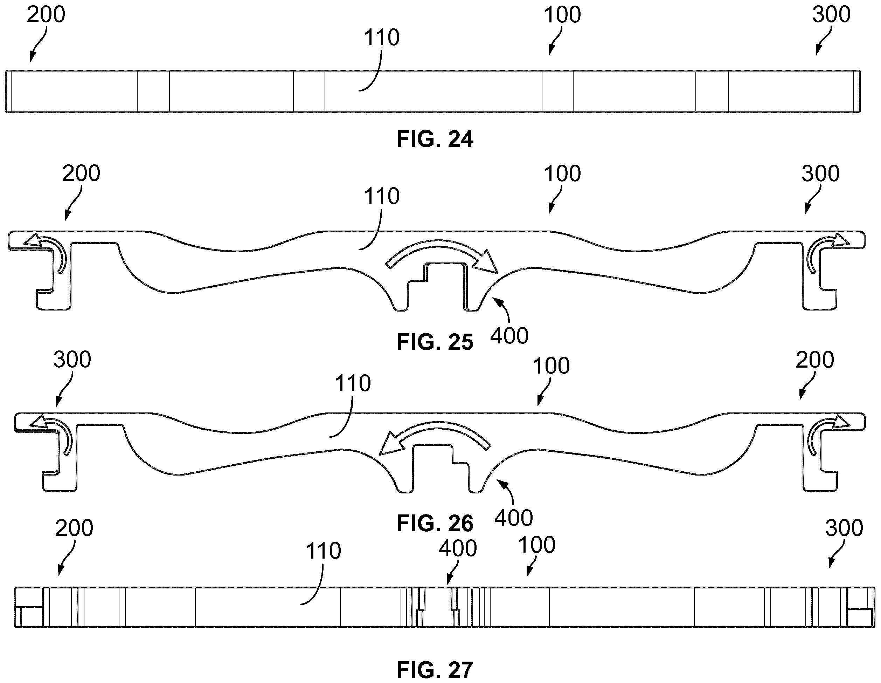

Figure 24 is a top view of the torque wrench 100 in accordance with an exemplary embodiment. Figure 25 is a front view of the torque wrench 100 in accordance with an exemplary embodiment. Figure 26 is a rear view of the torque wrench 100 in accordance with an exemplary embodiment. Figure 27 is a bottom view of the torque wrench 100 in accordance with an exemplary embodiment. Figures 24-27 illustrate the shaft 110, the first tightening socket 200, the second tightening socket 300, and the loosening socket 400.

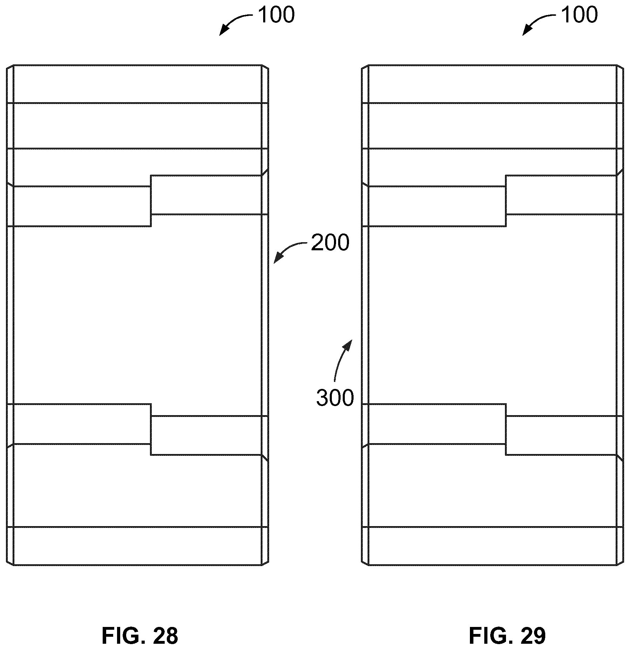

Figure 28 is a right side view of the torque wrench 100 in accordance with an exemplary embodiment. Figure 28 shows first tightening socket 200 at the right side of the torque wrench 100.

Figure 29 is a left view of the torque wrench 100 in accordance with an exemplary embodiment. Figure 29 shows the second tightening socket 300 at the left side of the torque wrench 100.

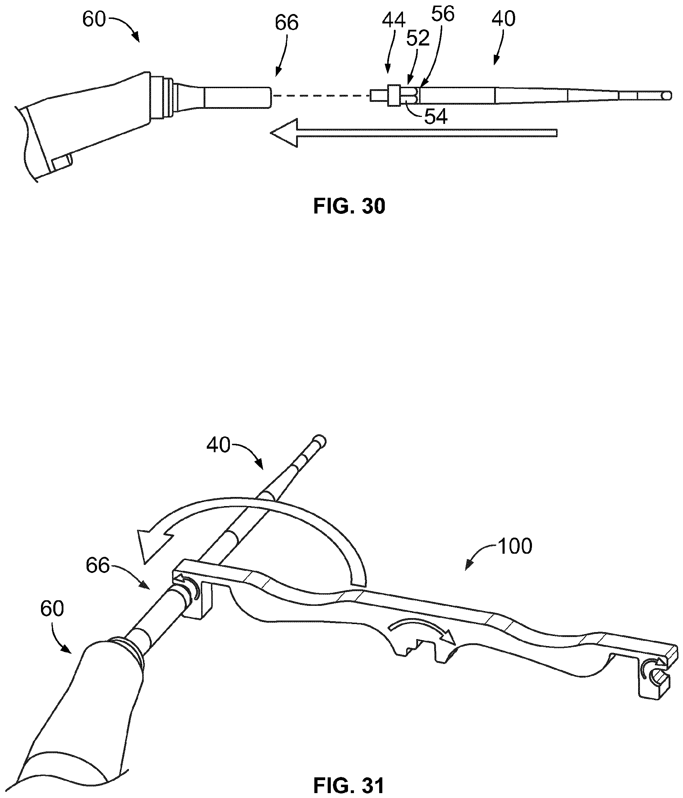

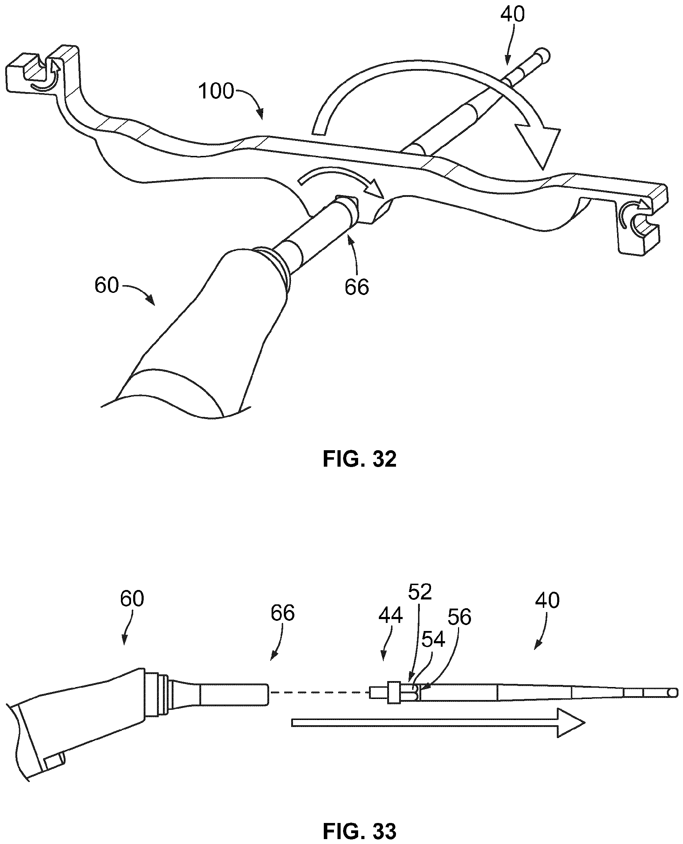

Figure 30 shows the first element 40 poised for loading onto the second element 60 in accordance with an exemplary embodiment. Figure 31 illustrates the torque wrench 100 in accordance with an exemplary embodiment for tightening the first element 40 to the second element 60. Figure 32 illustrates the torque wrench 100 in accordance with an exemplary embodiment for loosening the first element 40 from the second element 60. Figure 33 shows the first element 40 being removed from the second element 60 in accordance with an exemplary embodiment.

In the illustrated embodiment, the proximal end 44 of the first element 40 includes a threaded post configured to be threadably received in a threaded bore at the distal end 66 of the second element 60. The proximal end 44 of the first element 40 includes the drive elements 52 in the form of a hexagonal element having six flat surfaces 54 around the exterior of the first element 40. The keying feature 56 of the first element 40 is defined by a channel or groove along the side of the drive elements 52. The torque wrench 100 has features configured to interface with the drive elements and the keying feature 56 for proper tightening and loosening of the first element 40.

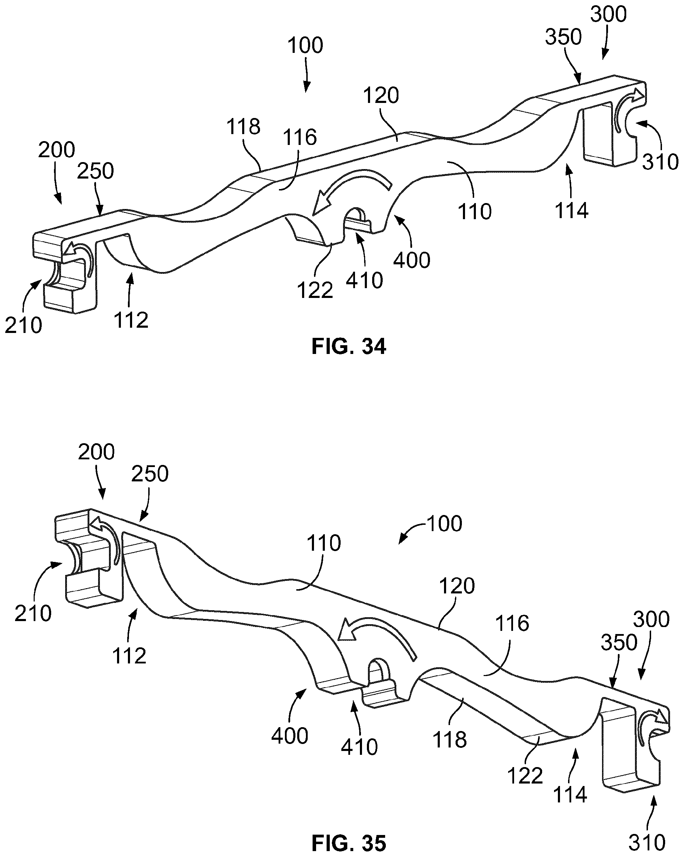

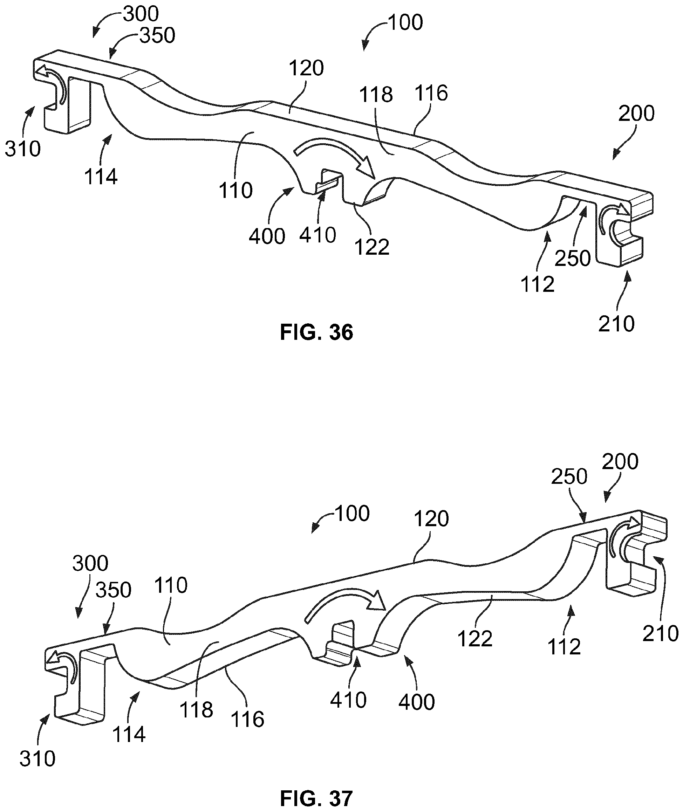

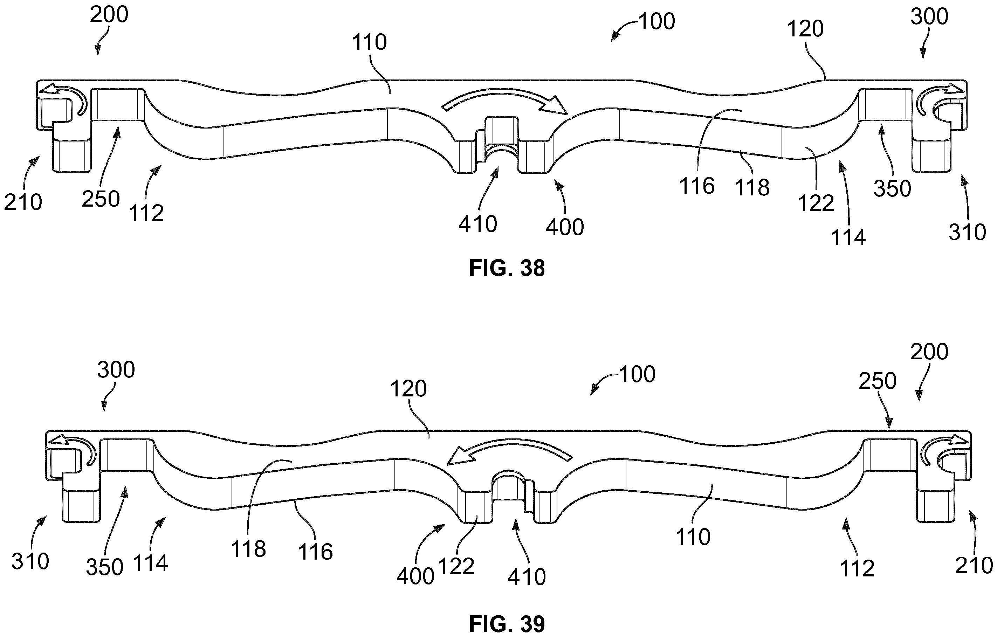

Figures 34 through 39 illustrate the torque wrench 100 in accordance with an exemplary embodiment. Figure 34 is a top perspective, front, right side view of the torque wrench 100. Figure 35 is a bottom perspective, front, right side view of the torque wrench 100. Figure 36 is a top perspective, rear, right side view of the torque wrench 100. Figure 37 is a bottom perspective, rear, right side view of the torque wrench 100. Figure 38 is a bottom perspective, rear view of the torque wrench 100. Figure 38 is a bottom perspective, front view of the torque wrench 100. The torque wrench 100 includes the shaft 110, the first tightening socket 200, the second tightening socket 300, and the loosening socket 400.

The shaft 110 extends between the first end 112 at the right side and the second end 114 at the left side. The shaft 110 includes the front 116 and the rear 118. The shaft 110 includes the top 120 and the bottom 122. In an exemplary embodiment, the first tightening socket 200 is provided at the first end 112, the second tightening socket 300 is provided at the second end 114, and the loosening socket 400 is provided at the bottom 122. Other locations are possible in alternative embodiments.

The first tightening socket 200 includes the first spanner pocket 210 configured to receive the first element 40 to tighten the first element 40 when the torque wrench 100 is rotated in a tightening direction. The first tightening socket 200 is connected to the first end 112 of the shaft 110 by the first weakened section 250 configured to yield at the predetermined torque. The first weakened section 250 is located between the first spanner pocket 210 and the first end 112 of the shaft 110.

The second tightening socket 300 includes the second spanner pocket 310 configured to receive the first element 40 to tighten the first element 40 when the torque wrench 100 is rotated in a tightening direction. The second tightening socket 300 is connected to the second end 114 of the shaft 110 by a second weakened section 350 configured to yield at the predetermined torque. The second weakened section 350 is located between the second spanner pocket 310 and the second end 114 of the shaft 110.

The loosening socket 400 is separate and discrete from the first tightening socket 200 and the second tightening socket 300. For example, the loosening socket 400 is located remote from the first tightening socket 200 and from the second tightening socket 300. The loosening socket 400 is configured to receive the first element 40 during a loosening operation. The loosening socket 400 includes the loosening spanner pocket 410 configured to receive the first element 40 to loosen the first element 40 from the second element 60 when the torque wrench 100 is rotated in a loosening direction.

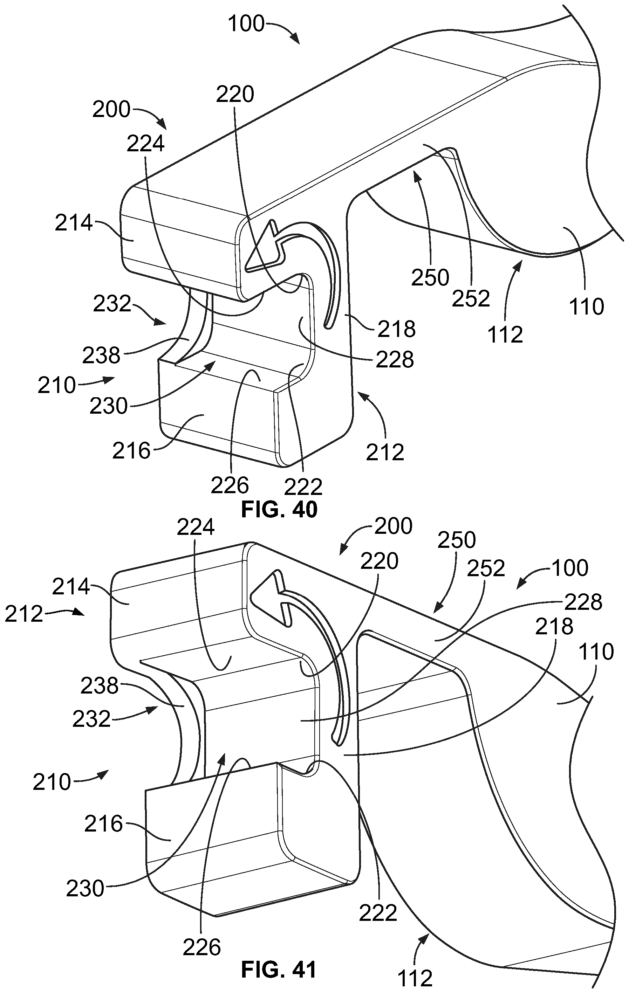

Figure 40 is a top perspective view of a portion of the torque wrench 100 showing the first tightening socket 200 in accordance with an exemplary embodiment. Figure 41 is a bottom perspective view of a portion of the torque wrench 100 showing the first tightening socket 200 in accordance with an exemplary embodiment. The first tightening socket 200 includes the first spanner pocket 210 and the first weakened section 250 connecting the first spanner pocket 210 to the first end 112 of the shaft 110.

The first tightening socket 200 includes the first head 212 forming the first spanner pocket 210. The first head 212 includes the upper jaw 214, the lower jaw 216, and the spanner wall 218 between the upper jaw 214 and the lower jaw 216. The spanner wall 218 meets the upper jaw 214 at the upper corner 220 and the spanner wall 218 meets the lower jaw 216 at the lower corner 222. The upper jaw 214, the lower jaw 216, and the spanner wall 218 include interface surfaces 224, 226, 228, respectively, at the interior surfaces that form the receiving pocket 230 for receiving the first element 40. In an exemplary embodiment, the interface surfaces 224, 226, 228 include flat surfaces configured to interface with the first element 40 to rotate the first element 40 in the tightening direction.

In an exemplary embodiment, the first tightening socket 200 includes a keying feature 238 at the rear for keyed mating with the first element 40. The keying feature 238 is in the form of a protrusion or tab protruding into the receiving pocket 230. In an exemplary embodiment, the keying feature 238 includes a semi-circular opening or cutout configured to receive a portion of the first element 40, such as the cylindrical exterior surface of the first element 40. The keying feature 238 may include fillets in the corners 220, 222. The keying feature 238 may extend along the upper jaw 214 and/or the lower jaw 216 and/or the spanner wall 218. The keyed mating with the first element 40 ensures that the first tightening socket 200 is mated with the first element 40 in only one orientation to ensure that the first element 40 is rotated in the correct direction when the torque wrench 100 is used to tighten the first element 40. The keying features 232 may be provided at other locations in alternative embodiments. In an exemplary embodiment, the keying feature 238 is arranged to prevent the torque wrench 100 from being coupled to the first element 40 in an incorrect orientation (for example, upside down).

The first weakened section 250 extends between the first spanner pocket 210 and the first end 112 of the shaft 110. The first weakened section 250 includes the first neck 252 located between the shaft 110 and the first head 212. The first neck 252 is located remote from the receiving pocket 230 of the first spanner pocket 210 such that the first weakened section 250 may be spaced apart from the first element 40. The neck 252 of the first weakened section 250 has a reduced cross-sectional area compared to the shaft 110. The neck 252 is weakened compared to the shaft 110 to define the area of deformation when the torque limit of the first tightening socket 200 is reached. For example, when the predetermined peak torque is reached during the tightening process, the first weakened section 250 starts to bend and deform rather than transfer the rotation of the shaft 110 to the first element 40. The torque wrench 100 is manufactured from a ductile material, such as an aluminum material, allowing for plastic deformation when the predetermined torque limit is reached. The first weakened section 250 may consist of a material with a smaller stiffness compared to the material of the shaft 110.

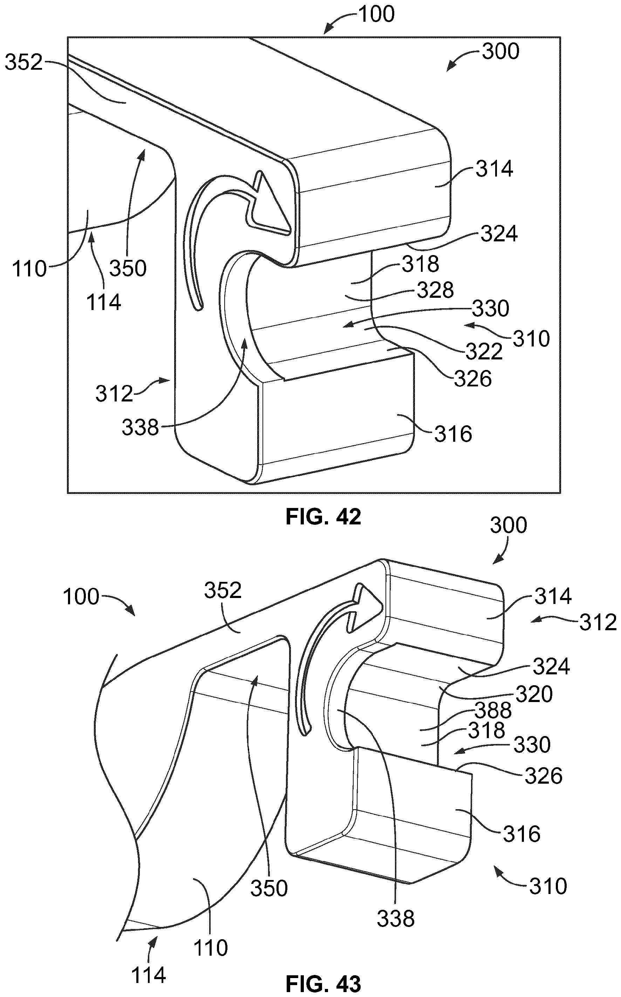

Figure 42 is a bottom perspective view of a portion of the torque wrench 100 showing the second tightening socket 300 in accordance with an exemplary embodiment. Figure 43 is a top perspective view of a portion of the torque wrench 100 showing the second tightening socket 300 in accordance with an exemplary embodiment. The second tightening socket 300 includes the second spanner pocket 310 and the second weakened section 350 connecting the second spanner pocket 310 to the second end 114 of the shaft 110.

The second tightening socket 300 includes the second head 312 forming the second spanner pocket 310. The second head 312 includes the upper jaw 314, the lower jaw 316, and the spanner wall 318 between the upper jaw 314 and the lower jaw 316. The spanner wall 318 meets the upper jaw 314 at the upper corner 320 and the spanner wall 318 meets the lower jaw 316 at the lower corner 322. The upper jaw 314, the lower jaw 316, and the spanner wall 318 include interface surfaces 324, 326, 328, respectively, at the interior surfaces that form the receiving pocket 330 for receiving the first element 40. In an exemplary embodiment, the interface surfaces 324, 326, 328 include flat surfaces configured to interface with the first element 40 to rotate the first element 40 in the tightening direction.

In an exemplary embodiment, the second tightening socket 300 includes a keying feature 338 at the rear for keyed mating with the first element 40. The keying feature 338 is in the form of a protrusion or tab protruding into the receiving pocket 330. In an exemplary embodiment, the keying feature 338 includes a semi-circular opening or cutout configured to receive a portion of the first element 40, such as the cylindrical exterior surface of the first element 40. The keying feature 338 may include fillets in the corners 320, 322. The keying feature 338 may extend along the upper jaw 314 and/or the lower jaw 316 and/or the spanner wall 318. The keyed mating with the first element 40 ensures that the second tightening socket 300 is mated with the first element 40 in only one orientation to ensure that the first element 40 is rotated in the correct direction when the torque wrench 100 is used to tighten the first element 40. The keying feature 338 may be provided at other locations in alternative embodiments. In an exemplary embodiment, the keying feature 338 is arranged to prevent the torque wrench 100 from being coupled to the first element 40 in an incorrect orientation (for example, upside down).

The second weakened section 350 extends between the second spanner pocket 310 and the second end 112 of the shaft 110. The second weakened section 350 includes the second neck 352 located between the shaft 110 and the second head 312. The second neck 352 is located remote from the receiving pocket 330 of the second spanner pocket 310 such that the second weakened section 350 may be spaced apart from the first element 40. The neck 352 of the second weakened section 350 has a reduced cross-sectional area compared to the shaft 110. The neck 352 is weakened compared to the shaft 110 to define the area of deformation when the torque limit of the second tightening socket 300 is reached. For example, when the predetermined peak torque is reached during the tightening process, the second weakened section 350 starts to bend and deform rather than transfer the rotation of the shaft 110 to the first element 40. The torque wrench 100 is manufactured from a ductile material, such as an aluminum material, allowing for plastic deformation when the predetermined torque limit is reached. The second weakened section 350 may consist of a material with a smaller stiffness compared to the material of the shaft 110.

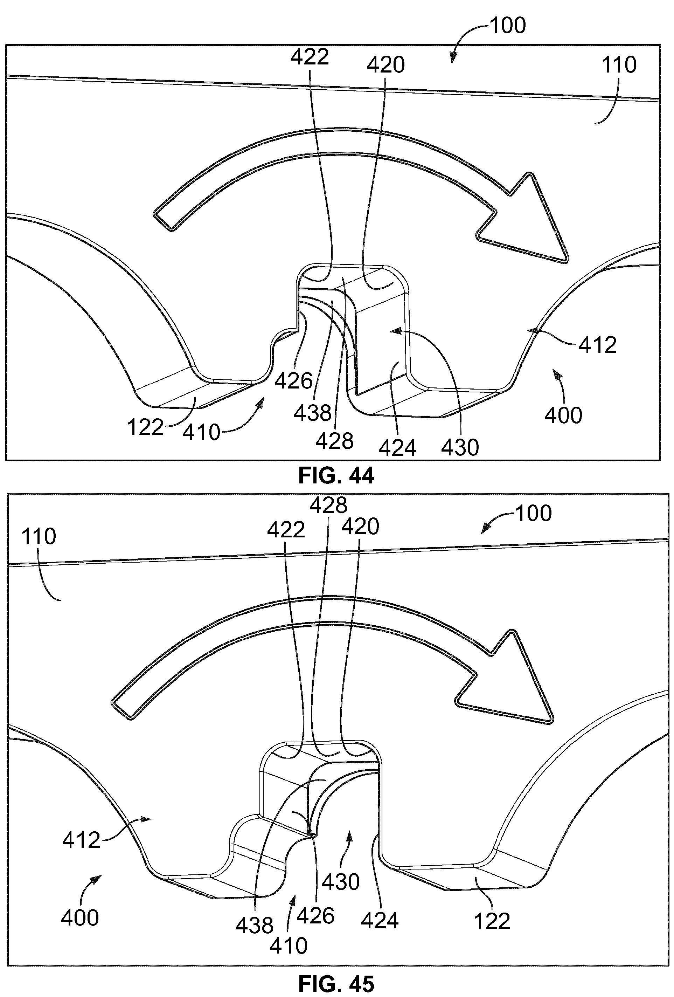

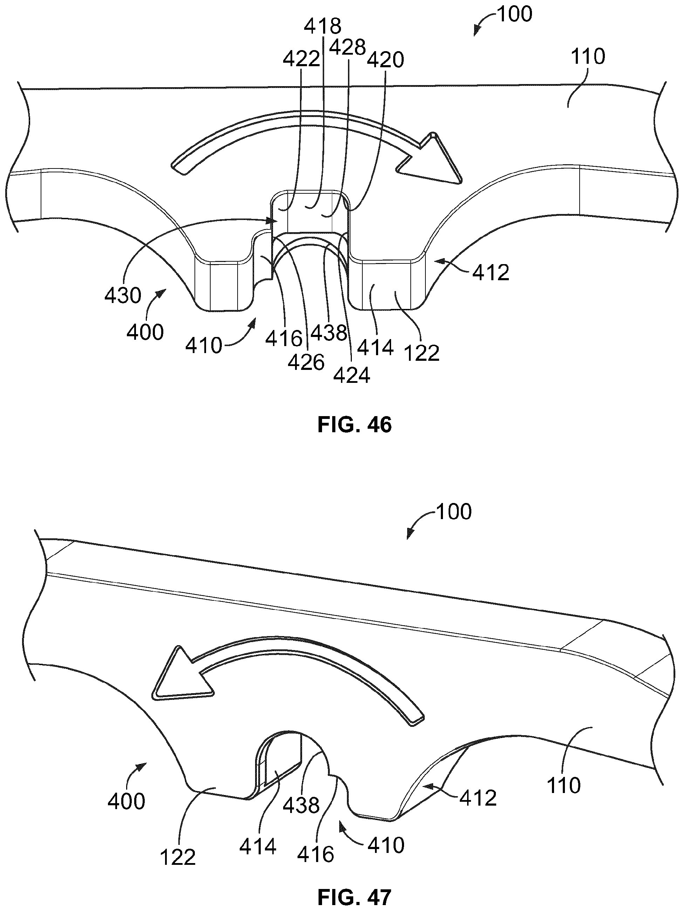

Figure 44 is a front perspective view of a portion of the torque wrench 100 from the right side showing the loosening socket 400 in accordance with an exemplary embodiment. Figure 45 is a front perspective view of a portion of the torque wrench 100 from the left side showing the loosening socket 400 in accordance with an exemplary embodiment. Figure 46 is a front perspective view of a portion of the torque wrench 100 showing the loosening socket 400 in accordance with an exemplary embodiment. Figure 47 is a rear perspective view of a portion of the torque wrench 100 showing the loosening socket 400 in accordance with an exemplary embodiment. The loosening socket 400 includes the loosening spanner pocket 410 at the bottom 122 of the shaft 110.

In an exemplary embodiment, the loosening socket 400 includes the loosening head 412 forming the loosening spanner pocket 410. The loosening head 412 includes the first jaw 414, the second jaw 416, and the spanner wall 418 between the first jaw 414 and the second jaw 416. The loosening spanner pocket 410 is bounded by the first jaw 414, the second jaw 416, and the spanner wall 418. In an exemplary embodiment, the spanner wall 418 meets the first jaw 414 at the first corner 420 and the spanner wall 418 meets the second jaw 416 at the second corner 422. The first jaw 414, the second jaw 416, and the spanner wall 418 include the interface surfaces 424, 426, 428, respectively, at the interior surfaces that form the receiving pocket 430 for receiving the first element 40. In an exemplary embodiment, the interface surfaces 424, 426, 428 include flat surfaces configured to interface with the first element 40 to rotate the first element 40 in the loosening direction.

In an exemplary embodiment, the loosening socket 400 includes a keying feature 438 at the rear for keyed mating with the first element 40. The keying feature 438 is in the form of a protrusion or tab protruding into the receiving pocket 430. In an exemplary embodiment, the keying feature 438 includes a semi-circular opening or cutout configured to receive a portion of the first element 40, such as the cylindrical exterior surface of the first element 40. The keying feature 438 may include fillets in the corners 420, 422. The keying feature 438 may extend along the first jaw 414 and/or the second jaw 416 and/or the spanner wall 418. The keyed mating with the first element 40 ensures that the loosening socket 400 is mated with the first element 40 in only one orientation to ensure that the first element 40 is rotated in the correct direction when the torque wrench 100 is used to loosen the first element 40. The keying feature 438 may be provided at other locations in alternative embodiments. In an exemplary embodiment, the keying feature 438 is arranged to prevent the torque wrench 100 from being coupled to the first element 40 in an incorrect orientation (for example, upside down).

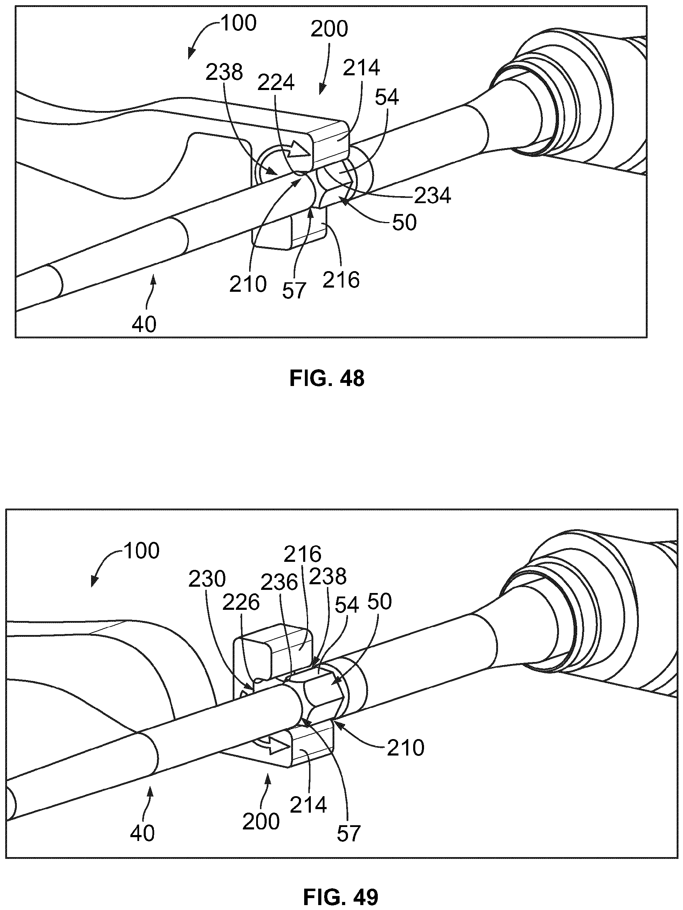

Figure 48 illustrates the torque wrench 100 with the first tightening socket 200 coupled to the first element 40 and configured to be tightened by rotating the torque wrench 100 in a tightening direction. Figure 49 illustrates the torque wrench 100 with the first tightening socket 200 incorrectly aligned with the first element 40 and unable to be coupled to the first element 40. Figure 49 shows the torque wrench 100 inverted 180° (for example, upside down) such that the first tightening socket 200 is unable to receive the first element 40. For example, the keying feature 238 prevents the first tightening socket 200 from coupling to the first element 40 in an incorrect orientation.

**In** an exemplary embodiment, the first element 40 includes a keying feature 57 at the torque wrench interface 50. When the torque wrench 100 is properly oriented relative to the first element 40, the interface surfaces 224, 226 of the upper and lower jaws 214, 216 are aligned with the flat surfaces 54 at the torque wrench interface 50. The interface surfaces 234, 236 are configured to seat on the flat surfaces 54 and are used to drive the first element 40 in the tightening direction when the torque wrench 100 is rotated in the tightening direction. The keying feature 238 is configured to interface with the keying feature 57 of the first element 40. For example, the wall defining the keying feature 238 is received in the groove or channel at the end of the torque wrench interface 50 defining the keying feature 57. Other types of keying features may be used in alternative embodiments. When the torque wrench 100 is improperly oriented (Figure 49), the first spanner pocket 210 is unable to receive the first element 40. The keying feature 238 bottoms out on the flat surfaces 54 preventing loading of the first element 40 into the receiving pocket 230.

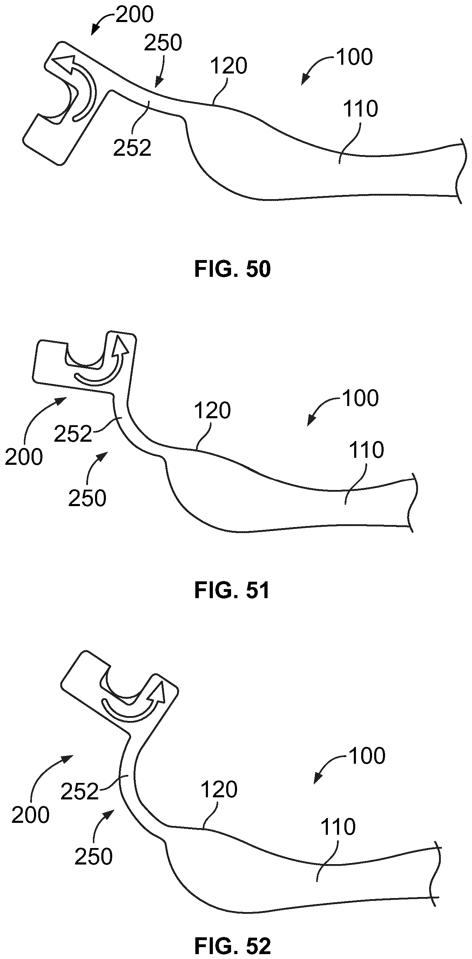

Figure 50 illustrates the torque wrench 100 in a failed (or yielded) state in accordance with an exemplary embodiment. Figure 51 illustrates the torque wrench 100 in a failed state in accordance with an exemplary embodiment. Figure 52 illustrates the torque wrench 100 in a failed state in accordance with an exemplary embodiment. Figures 50-52 shows the first weakened section 250 deformed. For example, the neck 252 is bent upward and is no longer planar. The first weakened section 250 is deformed during the tightening process when the torque limit is reached.

Figure 50 illustrates the first weakened section 250 deflected approximately 15°. Figure 51 illustrates the first weakened section 250 deflected approximately 90°. Figure 52 illustrates the first weakened section 250 deflected approximately 120°. The amount of deformation depends on the amount of rotation of the torque wrench 100 in the tightening direction after the peak torque is reached. The amount of deformation may be in the range of between approximately 0° and 180°. However, the amount of deformation may depend on the starting orientation of the first tightening socket 200 relative to the shaft 110. The amount of deformation may be limited by the shaft 110. For example, the first tightening socket 200 may be deformed until the first tightening socket 200 bottoms out against the top 120 of the shaft 110. Optionally, the first tightening socket 200 may have multiple uses. For example, the first tightening socket 200 may be used during a first tightening operation and deformed to the state shown in Figure 50, then may be used during a second tightening operation and deformed to the state shown in Figure 51, then may be used during a third tightening operation and deformed to the state shown in Figure 52. For example, the first weekend section 250 has a consistent peak torque regardless of the starting orientation.

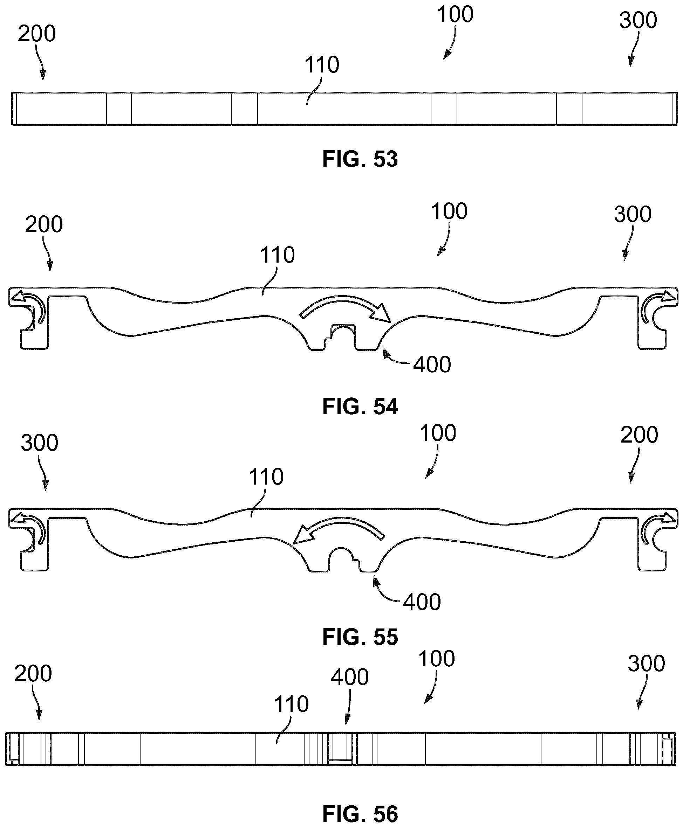

Figure 53 is a top view of the torque wrench 100 in accordance with an exemplary embodiment. Figure 54 is a front view of the torque wrench 100 in accordance with an exemplary embodiment. Figure 55 is a rear view of the torque wrench 100 in accordance with an exemplary embodiment. Figure 56 is a bottom view of the torque wrench 100 in accordance with an exemplary embodiment. Figures 53-56 illustrate the shaft 110, the first tightening socket 200, the second tightening socket 300, and the loosening socket 400.

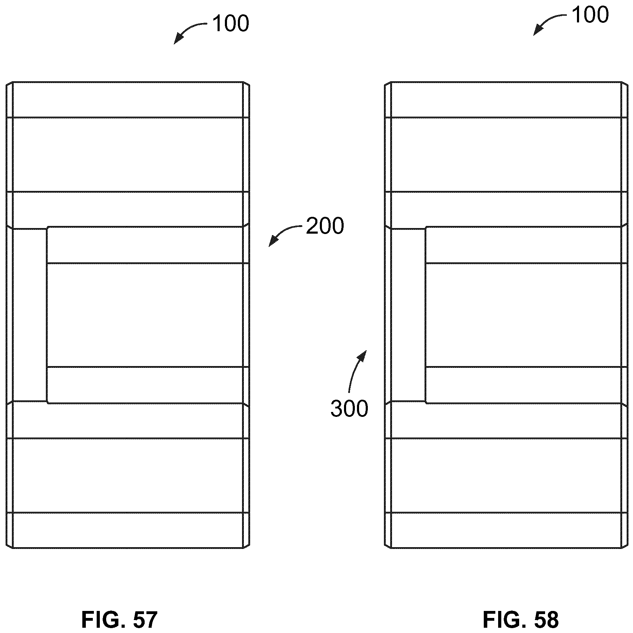

Figure 57 is a right side view of the torque wrench 100 in accordance with an exemplary embodiment. Figure 57 shows first tightening socket 200 at the right side of the torque wrench 100.

Figure 58 is a left view of the torque wrench 100 in accordance with an exemplary embodiment. Figure 58 shows the second tightening socket 300 at the left side of the torque wrench 100.

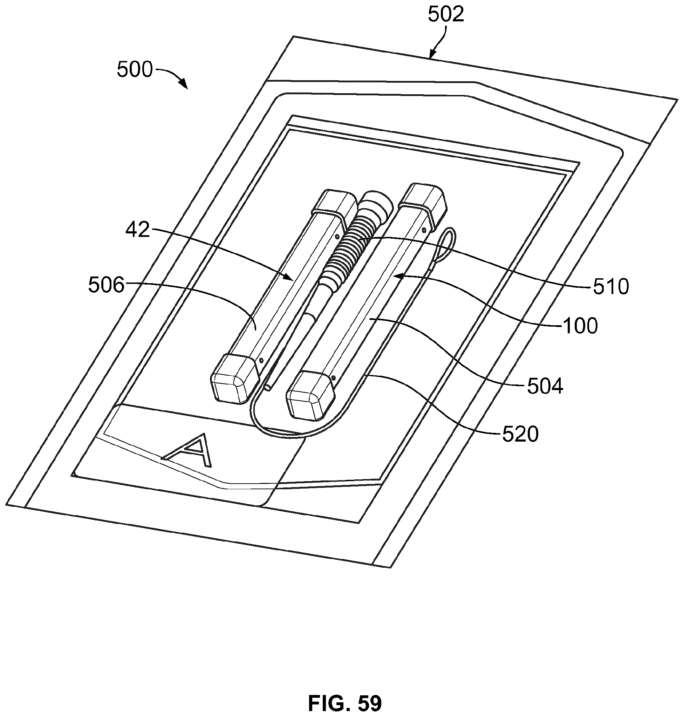

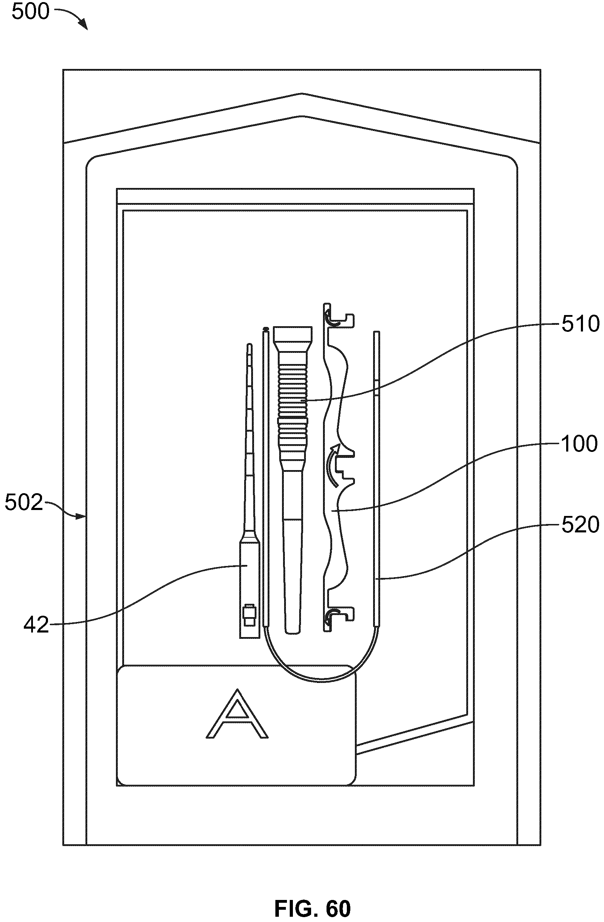

Figure 59 is a perspective view of a surgical device kit 500 in accordance with an exemplary embodiment. Figure 60 is a top view of the surgical device kit 500 in accordance with an exemplary embodiment. The surgical device kit 500 includes a package 502 holding the torque wrench 100. The torque wrench 100 may be contained within a container 504 (Figure 59). The surgical device kit 500 includes the first element, such as the surgical attachment 42, in the package 502. The surgical attachment 42 may be contained within a container 506 (Figure 59). Other components may be packaged in the package 502 with the torque wrench 100 and the surgical attachment 42, such as a sleeve 510 configured to be coupled to the handpiece to cover the surgical attachment 42, and a stylet 520. Other components may be arranged in the package 502. The components may be disposable. The components may be sterilizable.

It is be understood that the above description is intended to be illustrative, and not restrictive. For example, the above-described embodiments (and/or aspects thereof) may be used in combination with each other. In addition, many modifications may be made to adapt a particular situation or material to the teachings of the invention without departing from its scope. Dimensions, types of materials, orientations of the various components, and the number and positions of the various components described herein are intended to define parameters of certain embodiments, and are by no means limiting and are merely exemplary embodiments. Many other embodiments and modifications within the spirit and scope of the claims will be apparent to those of skill in the art upon reviewing the above description. The scope of the invention should, therefore, be determined with reference to the appended claims, along with the full scope of equivalents to which such claims are entitled. In the appended claims, the terms "including" and "in which" are used as the plain-English equivalents of the respective terms "comprising" and "wherein." Moreover, in the following claims, the terms "first," "second," and "third," etc. are used merely as labels, and are not intended to impose numerical requirements on their objects. Further, the limitations of the following claims are not written in means-plus-function format and are not intended to be interpreted based on 35 U.S.C. § 112(f), unless and until such claim limitations expressly use the phrase "means for" followed by a statement of function void of further structure.
The following introduces independently claimable aspects of the invention as a series of clauses, which clauses may be represented by corresponding claims.
Clause 1. A torque wrench for connecting a first element to a second element, the torque wrench comprising:
   a shaft extending between a first end and a second end;
   a first tightening socket at the first end, the first tightening socket including a first spanner pocket configured to receive the first element to tighten the first element to the second element when the torque wrench is rotated in a tightening direction, the first tightening socket connected to the first end of the shaft by a first weakened section configured to yield at a predetermined peak torque; and
   a loosening socket separate from the first tightening socket, the loosening socket including a loosening spanner pocket configured to receive the first element to loosen the first element from the second element when the torque wrench is rotated in a loosening direction. Clause 2: The torque wrench of clause 1, wherein the first weakened section is spaced apart from the first element.
Clause 3: A torque wrench for connecting a first element to a second element, the torque wrench comprising:
   a shaft extending between a first end and a second end;
   a first tightening socket including a first spanner pocket configured to receive the first element to tighten the first element to the second element when the torque wrench is rotated in a tightening direction, the first tightening socket connected to the first end of the shaft by a first weakened section configured to yield at a predetermined peak torque; and
   a second tightening socket including a second spanner pocket configured to receive the first element to tighten the first element to the second element when the torque wrench is rotated in a tightening direction, the second tightening socket connected to the second end of the shaft by a second weakened section configured to yield at the predetermined peak torque.
Clause 4: The torque wrench of clause 3, wherein the first tightening socket and the second tightening socket are identical and face in opposite directions on the opposite first and second ends of the shaft.
Clause 5: The torque wrench of one of clause 3 or 4, wherein the torque wrench is a dual use disposable torque wrench with the first tightening socket being a single use torque socket unusable after the first weakened section yields and with the second tightening socket being a single use torque socket being unusable after the second weakened section yields.
Clause 6: The torque wrench of one of clauses 3 to 5, wherein the first tightening socket and the second tightening socket are used in different tightening operations to tighten the first element to the second element at different times.
Clause 7: The torque wrench of one of clauses 3 to 6, wherein the first weakened section includes a first neck having a reduced cross-sectional area compared to the shaft and/or the second weakened section including a second neck having a reduced cross-sectional area compared to the shaft, wherein preferably the reduced cross-sectional area of the second neck is equivalent to the reduced cross-sectional area of the first neck.
Clause 8: The torque wrench of one of clauses 3 to 7, wherein the first tightening socket includes an upper jaw and a lower jaw on opposite sides of the first spanner pocket, the upper jaw and the lower jaw of the first tightening socket having flat interface surfaces configured to engage flat interface surfaces of the first element to rotate the first element when the torque wrench is tightened, and/or wherein the second tightening socket includes an upper jaw and a lower jaw on opposite sides of the second spanner pocket, the upper jaw and the lower jaw of the second tightening socket having flat interface surfaces configured to engage flat interface surfaces of the first element to rotate the first element when the torque wrench is tightened.
Clause 9: The torque wrench of one of clauses 3 to 8, wherein the first tightening socket includes a first keying feature configured to interface with the first element in only one orientation, and/or wherein the second tightening socket including a second keying feature configured to interface with the first element in only one orientation.
Clause 10: The torque wrench of one of clauses 3 to 9, further comprising a loosening socket along the shaft, the loosening socket including a loosening spanner pocket configured to receive the first element to loosen the first element from the second element when the torque wrench is rotated in a loosening direction.
Clause 11: A method of attaching a first element to a second element comprising:
   providing a torque wrench including a shaft extending between a first end and a second end, a first tightening socket at the first end, and a loosening socket separate from the first tightening socket, the first tightening socket including a first spanner pocket configured to receive the first element, the first tightening socket connected to the first end of the shaft by a first weakened section configured to yield at a predetermined peak torque, the loosening socket including a loosening spanner pocket configured to receive the first element;
   coupling the first tightening socket to the first element;
   rotating the torque wrench in a tightening direction to tighten the first element to the second element using the first tightening socket, wherein the torque wrench yields at the first weakening section when the first element is tightened to the predetermined peak torque;
   coupling the loosening socket to the first element; and
   rotating the torque wrench in a loosening direction to loosen the first element from the second element using the loosening socket.
Clause 12: The method of clause 11, wherein said coupling the first tightening socket to the first element includes aligning a first keyed interface of the first tightening socket with a keyed interface of the first element.
Clause 13: The method of one of clauses 11 or 12, wherein the first tightening socket includes an upper jaw and a lower jaw on opposite sides of the first spanner pocket, the upper jaw and lower jaw of the first tightening socket having flat interface surfaces, said coupling the first tightening socket to the first element includes engaging the flat interface surfaces of the upper jaw and lower jaw to flat interface surfaces of the first element to rotate the first element when the torque wrench is tightened.
Clause 14: The method of one of clauses 11 to 13, wherein said providing a torque wrench includes providing the torque wrench including a second tightening socket at the second end of the shaft, the second tightening socket including a second spanner pocket configured to receive the first element to tighten the first element to the second element, the second tightening socket connected to the second end of the shaft by a second weakened section configured to yield at the predetermined peak torque, wherein the second tightening socket allows for a second tightening operation by the torque wrench prior to disposal of the torque wrench.
Clause 15: The method of clause 14, further comprising:
   coupling the second tightening socket to the first element; and
   rotating the torque wrench in a tightening direction to tighten the first element to the second element using the second tightening socket, wherein the torque wrench yields at the second weakening section when the first element is tightened to the predetermined peak torque.

## Claims

1. A torque wrench for connecting a first element to a second element, the torque wrench comprising:
a shaft extending between a first end and a second end;
a first tightening socket at the first end, the first tightening socket including a first spanner pocket configured to receive the first element to tighten the first element to the second element when the torque wrench is rotated in a tightening direction, the first tightening socket connected to the first end of the shaft by a first weakened section configured to yield at a predetermined peak torque; and
a loosening socket separate from the first tightening socket, the loosening socket including a loosening spanner pocket configured to receive the first element to loosen the first element from the second element when the torque wrench is rotated in a loosening direction.

2. The torque wrench of claim 1, further comprising a second tightening socket at the second end, the second tightening socket including a second spanner pocket configured to receive the first element to tighten the first element to the second element when the torque wrench is rotated in a tightening direction, the second tightening socket connected to the second end of the shaft by a second weakened section configured to yield at the predetermined peak torque.

3. The torque wrench of claim 2, wherein the first and second tightening sockets are identical and face in opposite directions on the opposite first and second ends of the shaft.

4. The torque wrench of one of claims 1 to 3, wherein the torque wrench is a disposable torque wrench with the first tightening socket and/or the second tightening socket being a single use torque socket unusable after the first weakened section and/or the second weakened section yield.

5. The torque wrench of one of claims 1 to 4, wherein the first weakened section deforms at the predetermined peak torque and/or by bending backwards toward the shaft.

6. The torque wrench of claim 5, wherein the first weakened section is configured to deform between a range of approximately 0° to approximately 180°, the applied torque plateauing at the predetermined peak torque through the range of deformation.

7. The torque wrench of one of claims 1 to 6, wherein the first weakened section includes a first neck having a reduced cross-sectional area compared to the shaft and/or extends between the first end of the shaft and the first tightening socket.

8. The torque wrench of claim 1 to 7, wherein the first tightening socket includes an upper jaw and a lower jaw on opposite sides of the first spanner pocket, the upper jaw and lower jaw of the first tightening socket having flat interface surfaces configured to engage flat interface surfaces of the first element to rotate the first element when the torque wrench is tightened.

9. The torque wrench of claim 8, wherein the lower jaw is shorter than the upper jaws allowing rotation of the first element in only the tightening direction.

10. The torque wrench of one of claims 1 to 9, wherein the first tightening socket and/or the second tightening socket include a first keying feature and/or a second keying feature configured to interface with the first element in only one orientation.

11. The torque wrench of one of claims 1 to 10, wherein the loosening socket includes a first jaw and a second jaw on opposite sides of the loosening spanner pocket, the first jaw and the second jaw having flat interface surfaces configured to engage flat interface surfaces of the first element to rotate the first element when the torque wrench is loosened.

12. The torque wrench of one of claims 1 to 11, wherein the first tightening socket and/or the second tightening socket faces in a first direction and the loosening socket faces in a second direction perpendicular to the first direction.

13. The torque wrench of one of claims 1 to 12, wherein the first tightening socket and/or the second tightening socket and the loosening socket are integral with the shaft to define a single piece torque wrench.

14. The torque wrench of one of claims 1 to 13, wherein the shaft is manufactured from a metal material being sterilizable, biocompatible and/or disposable.

15. A surgical device kit comprising:
a surgical attachment extending between a proximal end and a distal end, the proximal end configured to be removably coupled to a handpiece, the distal end having a working element for performing a surgical operation, the surgical attachment including a torque wrench interface; and
a torque wrench configured to interface with the surgical attachment at the torque wrench interface for tightening the surgical attachment to the handpiece to a predetermined peak torque and for loosening the surgical attachment from the handpiece, the torque wrench including a shaft extending between a first end and a second end and a first tightening socket at the first end, the first tightening socket including a first spanner pocket configured to receive the surgical attachment at the torque wrench interface to tighten the surgical attachment to the handpiece when the torque wrench is rotated in a tightening direction, the first tightening socket connected to the first end of the shaft by a first weakened section configured to yield at the predetermined peak torque, and a loosening socket separate from the first tightening socket, the loosening socket including a loosening spanner pocket configured to receive the surgical attachment at the torque wrench interface to loosen the surgical attachment from the handpiece when the torque wrench is rotated in a loosening direction.

16. The surgical device kit of claim 15, the torque wrench further comprising a second tightening socket at the second end, the second tightening socket including a second spanner pocket configured to receive the first element to tighten the first element, the second tightening socket connected to the second end of the shaft by a second weakened section configured to yield at a predetermined peak torque.

17. The surgical device kit of one of claims 15 or 16, further comprising a sleeve configured to surround the surgical attachment, the sleeve configured to be coupled to the handpiece.

18. The surgical device kit of one of claims 15 to 17, wherein the first tightening socket and/or the second tightening socket include a keying feature configured to interface with the surgical instrument at the torque wrench interface in only one orientation.
